# EUROPÄISCHE PATENTSCHRIFT

(11) **EP 2 467 017 B1**
(45) Veröffentlichungstag und Bekanntmachung des Hinweises auf die Patenterteilung: **29.05.2013**
(21) Anmeldenummer: 09777976.3
(22) Anmeldetag: 19.08.2009
(51) Int. Cl.: A01M 1/20, A61L 9/12

(54) **VORRICHTUNG ZUR ABGABE VON FLÜCHTIGEN SUBSTANZEN, INSBESONDERE VON DUFTSTOFFEN UND/ODER INSEKTIZIDEN**
DEVICE FOR DISPENSING VOLATILE SUBSTANCES, IN PARTICULAR FRAGRANCES AND/OR INSECTICIDES
DISPOSITIF DE DIFFUSION DE SUBSTANCES VOLATILES, EN PARTICULIER DE PARFUMS ET/OU D'INSECTICIDES

(43) Veröffentlichungstag der Anmeldung: 27.06.2012
(73) Patentinhaber: CTR, Lda, 2135-301 Samora Correia (PT)
(72) Erfinder: VIEIRA, Pedro, Queiroz, 2750-004 Cascais (PT)
(74) Vertreter: Liebl, Thomas
(86) Internationale Anmeldenummer: PCT/EP2009/006011
(87) Internationale Veröffentlichungsnummer: WO 2011/020480

(56) Entgegenhaltungen:
- WO-A1-02/100449
- WO-A1-2009/018862
- GB-A- 2 298 793
- GB-A- 2 333 233
- US-A1- 2007 217 771

## Beschreibung

Die Erfindung betrifft eine Vorrichtung zur Abgabe von flüchtigen Substanzen, insbesondere von Duftstoffen und/oder Insektiziden, nach dem Oberbegriff des Anspruchs 1, siehe z.B. document GB-A-2 298 793.

Derartige Vorrichtungen zur Abgabe von flüchtigen Substanzen, insbesondere von Duftstoffen und/oder Insektiziden, sind allgemein bekannt, zum Beispiel in Verbindung mit eine Heizeinrichtung aufweisenden Verdampfungsvorrichtungen, bei denen ein mit Flüssigkeit vollgesaugter Docht zur Erhöhung der Verdampfungsrate in eine Ausnehmung oder Ausbuchtung eines Heizblocks der Verdampfungsvorrichtung einragt.

Demgegenüber ist es Aufgabe der vorliegenden Erfindung, eine alternative Vorrichtung zur Abgabe von flüchtigen Substanzen, insbesondere von Duftstoffen und/oder Insektiziden, zu schaffen, mit der eine effektive Wirkstoff- bzw. Substanzabgabe an die Umgebung auf baulich einfache, funktionssichere sowie bedienerfreundliche Weise möglich ist.

Diese Aufgabe wird gelöst mit den Merkmalen des unabhängigen Patentanspruchs 1. Vorteilhafte Ausgestaltungen hierzu sind Gegenstand der darauf rückbezogenen Unteransprüche.

Erfindungsgemäß weist eine Vorrichtung zur Abgabe von flüchtigen Substanzen, insbesondere von Duftstoffen und/oder Insektiziden, wenigstens ein die wenigstens eine abzugebende Substanz aufweisendes, insbesondere zwischenspeicherndes Speichermedium auf, wobei wenigstens ein Mittel zum Erzeugen wenigstens eines Luftstroms vorgesehen ist, welcher wenigstens eine Luftstrom so auf das wenigstens eine Speichermedium gelenkt ist, dass dieser die Abgaberate der abzugebenden Substanz an die Umgebung gegenüber dem nicht-angeströmten Zustand erhöht.

Erfindungsgemäß ist hierbei vorgesehen, dass der wenigstens eine von dem Mittel zum Erzeugen wenigstens eines Luftstroms erzeugte Luftstrom das Speichermedium in einem definierten Bereich um- und/oder anströmt.

Mit einer derartigen erfindungsgemäßen Lösung kann insbesondere die Abgaberate bzw. "Verdampfungsrate" von solchen Speichermedien erhöht werden, bei denen wenigstens eine Flüssigkeit und/oder wenigstens ein Gel über eine Art Membran aus dem Speichermedium in die Umgebung diffundiert. Denn durch das Anströmen eines derartigen Speichermediums mittels eines definierten Luftstroms wird die Substanzabgabe bzw. die Diffusion der abzugebenden Substanzen durch die Membran beschleunigt, was wiederum die Produkteffizienz wesentlich erhöht.

Der Begriff "abzugebende Substanz" ist hier ausdrücklich in einem weiten Sinne zu verstehen und umfasst sämtliche Wirkstoffe, die in die Umgebung verdampft bzw. verflüchtigt werden können, zum Beispiel Duftstoffe und/oder Insektizide.

Das wenigstens eine Mittel zum Erzeugen wenigstens eines Luftstroms ist bevorzugt durch eine Lüfter- und/oder Gebläse- und/oder Ventilatoranordnung gebildet, die zum Beispiel wenigstens ein Laufrad und/oder ein propellerartiges Flügelrad aufweist. Grundsätzlich können jedoch auch alle anderen geeigneten Mittel zum Erzeugen wenigstens eines Luftstroms verwendet werden. Nachfolgend wird die Erfindung lediglich aus Übersichtlichkeitsgründen stets in Verbindung mit dem Begriff Ventilatoranordnung als konkretem Mittel zum Erzeugen wenigstens eines Luftstroms beschrieben, ohne dass dies als Einschränkung auf eine Ventilatoranordnung verstanden werden soll. Vielmehr ist die Begrifflichkeit Ventilatoranordnung hier in einem umfassenden Sinne zu verstehen.

Erfindungsgemäß ist das wenigstens eine Mittel (9) zum Erzeugen wenigstens eines Luftstroms Bestandteil einer Andockstation, mit der das wenigstens eine Speichermedium in einem definierten Andockbereich mittelbar über ein das wenigstens eine Speichermedium haltendes und/oder aufnehmendes Bauteil, d.h. eine nachfolgend noch näher beschriebene Halte- und/oder Aufnahmeeinrichtung, mittels der das wenigstens eine Speichermedium gehaltert wird, und/oder unmittelbar lösbar verbindbar ist.

Eine derartige Andockstation weist somit eine mit einem Ladegerät für zum Beispiel elektrische Geräte, wie zum Beispiel Telefone oder dergleichen, vergleichbare Funktion auf, in die bzw. an die das jeweilige Speichermedium einfachst angedockt wird. Bevorzugt ist dabei vorgesehen, dass, zum Beispiel in Verbindung mit einem Set, an eine einzige Andockstation mehrere unterschiedliche, wenigstens ein Speichermedium halternde und/oder aufnehmende und nachfolgend noch näher beschriebene Halte- und/oder Aufnahmeeinrichtungen benutzerindividuell und/oder je nach den gewünschten konkreten Anwendungs- und Verwendungswünschen angedockt werden können. Beispielsweise können sich die Halte- und/oder Aufnahmeeinrichtungen hinsichtlich der Form und/oder hinsichtlich der Muster von Lufteinlassöffnungen unterscheiden. Ebenso können sich die Halte- und/oder Aufnahmeeinrichtungen aber auch hinsichtlich der Anzahl und der Art und Weise der darin aufgenommenen Speichermedien unterscheiden.

In Verbindung mit einer derartigen Andockstation weist, diese Andockstation ein ein- oder mehrteiliges Gehäuse auf, in dem das wenigstens eine Mittel (9) zur Erzeugung wenigstens eines Luftstroms aufgenommen ist. Dadurch ist zum einen ein Berührschutz gegeben und kann zum anderen über zum Beispiel definierte Luftauslassöffnungsgeometrien eine definierte Luftströmung eingestellt werden. Das heißt, dass das Gehäuse wenigstens eine diesen Luftauslass ermöglichende Luftauslassöffnung aufweist.

Grundsätzlich kann diese Luftauslassöffnung auch als Lufteinlassöffnung fungieren, wobei es jedoch für eine gute Funktionsfähigkeit besonders vorteilhaft ist, die wenigstens eine Lufteinlassöffnung separat und unabhängig von der wenigstens einen Luftauslassöffnung an einer geeigneten Stelle am Gehäuse vorzusehen. Hierbei ist es besonders vorteilhaft, wenn die Ventilatoranordnung, insbesondere deren Laufrad und/oder Flügelrad, unmittelbar der wenigstens einen Luftauslassöffnung zugeordnet ist, zum Beispiel in Verbindung mit einer die wenigstens eine Luftauslassöffnung aufweisenden Gehäuseoberseite unmittelbar unterhalb dieser im Gehäuse angeordnet ist, während die wenigstens eine Lufteinlassöffnung bevorzugt beabstandet dazu im mehr seitlichen Gehäusebereich angeordnet ist, zum Beispiel an einer Seitenwand und/oder im Übergangsbereich zwischen einer Seitenwand und einer Gehäuseoberseite. Dadurch ist sichergestellt, dass der von der Ventilatoranordnung erzeugte wenigstens eine Luftstrom bevorzugt mehr oder weniger direkt auf die wenigstens eine Luftauslassöffnung gerichtet ist, wodurch wiederum die gewünschten Strömungsverhältnisse auf einfache und zuverlässige Weise eingestellt werden können. Grundsätzlich wäre aber auch eine mittelbare Luftzuführung von der Ventilatoranordnung zur wenigstens einen Luftauslassöffnung denkbar, zum Beispiel über einen Strömungskanal oder dergleichen.

Besonders bevorzugt ist hierbei eine konkrete Ausgestaltung, bei der die Andockstation als Standgerät mit einer Aufstandsfläche und/oder einer Anschlussfläche für das wenigstens eine Speichermedium bzw. die dieses gegebenenfalls halternde und/oder aufnehmende Bauteil ausgebildet ist, um dieses auf einer Oberfläche abstellen zu können, zum Beispiel auf einem Tisch und/oder auf dem Boden und/oder in einem Regal. Die Anschlussfläche wird dabei bevorzugt durch eine nach oben weisende Seitenfläche (Oberfläche) der Andockstation gebildet, von der das wenigstens eine angedockte Speichermedium bzw. das das wenigstens eine Speichermedium aufnehmende Bauteil in eine definierte Raumrichtung, bevorzugt in Hochachsenrichtung, turmartig oder kastenartig oder blockartig wegragt.

Mit einer derartigen als Andockstation ausgebildeten Vorrichtung zur Abgabe von flüchtigen Substanzen ergibt sich somit eine völlig neuartige Konzeption derartiger "Verdampfungs"-Vorrichtungen, die individuell ausgestaltbar und einsetzbar sind und zudem sehr funktionssicher und bedienerfreundlich sind. Denn einem Bediener erschließt sich die technische Funktionsweise eines derartigen Konstruktionsprinzipes, das er von zum Beispiel Ladegeräten für elektronische Bauteile kennt, sofort und ohne weiteres, was wiederum zu einer hohen Kundenakzeptanz führt.

Um sicherzustellen, dass eine ausreichende Menge des Luftstroms zu dem wenigstens einen Speichermedium strömen kann, ist erfindungsgemäß vorgesehen, dass das Gehäuse der Andockstation an einer dem wenigstens einen Speichermedium zugewandten Gehäuseseite wenigstens eine Luftauslassöffnung aufweist, insbesondere wenigstens einen Luftauslassschlitz als Luftauslassöffnung aufweist. Insbesondere in Verbindung mit einer derartigen Ausgestaltung ist es vorteilhaft, dass die wenigstens eine Luftaustassöffnung und/oder das wenigstens eine Speichermedium so angeordnet und/oder einander so zugeordnet sind, dass der wenigstens eine Luftstrom das wenigstens eine Speichermedium in einem definierten Maße an- und/oder umströmt. Erfindungsgemäß ist hierbei eine konkrete Ausgestaltung, bei der der wenigstens eine Luftstrom das Speichermedium entlang einer definierten Anströmfläche des Speichermediums an- und/oder umströmt. Eine derartige definierte Anströmfläche kann zum Beispiel durch den zuvor bereits beschriebenen Membranbereich ausgebildet sein.

Wie bereits zuvor des Öfteren angedeutet, ist erfindungsgemäß vorgesehen, dass das wenigstens eine Speichermedium in einer Halte- und/oder Aufnahmeeinrichtung aufgenommen bzw. an einer solchen gehaltert ist, die mit der Andockstation in einem Andockbereich verbunden, insbesondere lösbar verbunden werden kann, bevorzugt von oben her aufgesetzt und/oder angedockt werden kann. Die Begrifflichkeit Halte-und/oder Aufnahmeeinrichtung ist hier ausdrücklich in einem umfassenden Sinne zu verstehen und soll jede Möglichkeit der Halterung des wenigstens einen Speichermediums umfassen und nicht nur die, wenngleich auch bevorzugte, Aufnahme des wenigstens einen Speichermediums in einem Aufnahmeraum und damit im Inneren der Halte- und/oder Aufnahmeeinrichtung.

Mit einer derartigen Halte- und/oder Aufnahmeeinrichtung ergeben sich die zuvor beschriebenen Vorteile, insbesondere hinsichtlich einer funktionssicheren Positionierung und Halterung der jeweiligen Speichermedien in der Vorrichtung bzw. im Gerät in Verbindung mit unterschiedlichsten Gestaltungsmöglichkeiten, um unterschiedlichste Designanforderungen zufriedenzustellen. Selbstverständlich kann mit derartigen Halte-und/oder Aufnahmeeinrichtungen, zum Beispiel über die Art und Weise der Ausgestaltung der wenigstens einen Luftauslassöffnung auch sehr gut die zum wenigstens einen Speichermedium geführte Luftströmung beeinflusst werden, zum Beispiel hinsichtlich der Menge und des Volumens bzw. auch der Luftgeschwindigkeit. Dadurch ergeben sich somit individuellste Gestaltungsmöglichkeiten derartiger erfindungsgemäßer Vorrichtungen.

Insbesondere kommt mit einer derartigen Halte- und/oder Aufnahmeeinrichtung das neuartige technische Konzept einer Andockstation zum Tragen, indem zum Beispiel in einem ersten Schritt das wenigstens eine Speichermedium in die Halte- und/oder Aufnahmeeinrichtung eingesetzt wird und anschließend die so bestückte Halte-und/oder Aufnahmeeinrichtung bevorzugt von mehr oder weniger oben her an der Andockstation angedockt wird. Das zum Beispiel Einsetzen des wenigstens einen Speichermediums erfolgt dabei ebenfalls bevorzugt von oben her, das heißt an einem dem Andockbereich abgewandten Ende der Halte- und/oder Aufnahmeeinrichtung, in welches das wenigstens eine Speichermedium bevorzugt von oben her eingesetzt wird. Es versteht sich von selbst, dass das wenigstens eine Speichermedium auch erst nach dem Andocken der Halte- und/oder Aufnahmeeinrichtung an die Andockstation in die Halte- und/oder Aufnahmeeinrichtung eingesetzt werden kann.

Besonders bevorzugt ist hierbei vorgesehen, dass die lösbare Verbindung der Andockstation mit der Halte- und/oder Aufnahmeeinrichtung mittels wenigstens einer mechanischen Rast- und/oder Abstütz- und/oder Steckverbindung erfolgt, wobei hier eine form- und/oder reibschlüssige Verbindung bevorzugt ist. Beispielsweise ist gemäß einer konkreten Ausgestaltung an der Andockstation wenigstens ein andockstationsseitiges Rast- und/oder Abstütz- und/oder Steckelement angeordnet, das im angedockten verbundenen Zustand von Halte- und/oder Aufnahmeeinrichtung und Andockstation mit wenigstens einem halte- und/oder aufnahmeeinrichtungsseitigen Rast- und/oder Abstütz- und/oder Steckgegenelement so zusammenwirkt, dass die Halte- und/oder Aufnahmeeinrichtung mit einer definierten Haltekraft in und/oder an der Andockstation bzw. dem Andockbereich der Andockstation gehalten ist. Dies kann zum Beispiel konkret über einander zugeordnete Rastvorsprünge oder Rastnasen einerseits und Rastausnehmungen andererseits erfolgen.

Konkret weist die Halte- und/oder Aufnahmeeinrichtung in wenigstens einem definierten Außenwandbereich wenigstens eine Lufteinlassöffnung auf, über die wenigstens ein über wenigstens eine andockstationsseitige Luftauslassöffnung ausströmender Luftstrom von der Außenseite der Halte- und/oder Aufnahmeeinrichtung her in diese ein- und/oder gegebenenfalls ausströmen kann. Derartige Lufteinlassöffnungen bilden somit bevorzugt seitliche Lufteinlassöffnungen aus, was insbesondere mit solchen Aufbauten von Speichermedium und Halte- und/oder Aufnahmeeinrichtung von Vorteil ist, bei denen ein Membranbereich eines Speichermediums in unmittelbarer Nachbarschaft der jeweils vorgesehenen halte-und/oder aufnahmeeinrichtungsseitigen Lufteinlassöffnungen angeordnet, das heißt diesem zugewandt ist. Dadurch wird nämlich eine effiziente Substanzabgabe und damit Anreicherung der Strömungsluft mit den jeweils abzugebenden Substanzen möglich.

Besonders bevorzugt ist hierbei vorgesehen, dass die wenigstens eine Lufteinlassöffnung ein definiertes Öffnungsmuster ausbildet und/oder wenigstens 20%, bevorzugt wenigstens 30%, höchst bevorzugt wenigstes 40% der zugeordneten Speichermediumfläche freigibt, um ausreichende und technisch sinnvolle Strömungsverhältnisse zu erzielen.

Eine besonders effektive Anreicherung der Luftströmung mit den abzugebenden Substanzen erfolgt für den Fall, dass an der Andockstation mehrere, vorzugsweise voneinander beabstandete Luftauslassöffnungen, die insbesondere durch Luftaustassschlitze gebildet sind, im Andockbereich um die Halte- und/oder Aufnahmeeinrichtung herum angeordnet und/oder jeweils halte- und/oder aufnahmeeinrichtungsseitigen Lufteinlassöffnungen aufweisenden Halte- und/oder Aufnahmeeinrichtungs-Wandbereichen zugeordnet sind. Die Zuordnung erfolgt hier insbesondere so, dass die aus den andockstationsseitigen Luftauslassöffnungen austretenden Luftströme auf definierten, im Wesentlichen geradlinigen Strömungsweg zu den Lufteinlassöffnungen strömen.

Gemäß einer besonders bevorzugten konkreten Ausführungsvariante hierzu weist die Halte- und/oder Aufnahmeeinrichtung eine block- oder kastenartige Außenkontur mit auf gegenüberliegenden Außenwandseiten liegenden Lufteinlassöffnungen auf, wobei zudem im Andockungsbereich auf gegenüberliegenden Seiten der Halte-und/oder Aufnahmeeinrichtung, den die Lufteinlassöffnungen aufweisenden Wandseiten zugeordnet, wenigstens eine andockstationsseitige Luftauslassöffnung, die bevorzugt durch wenigstens einen Luftauslassschlitz gebildet ist, angeordnet ist. Hierdurch ergibt sich ein insgesamt kompakter Vorrichtungsaufbau, mit dem eine effektive Anreicherung der Umgebungsluft auf einfache Weise möglich wird.

Gemäß einer hierzu weiteren Erfindungsvariante kann vorgesehen sein, dass die Andockstation eine U-förmige Außenkontur mit U-Schenkeln aufweist, die die Halte-und/oder Aufnahmeeinrichtung mit den U-Schenkeln wenigstens bereichsweise formschlüssig umgreifen, insbesondere an den, den einander zugeordneten Lufteinlass- und Luftauslassöffnung abgewandten, gegenüberliegenden Außenwandseiten wenigstens bereichsweise formschlüssig umgreifen. Dadurch wird eine noch bessere Abstützung und Halterung der Halte- und/oder Aufnahmeeinrichtung an der Andockstation erzielt.

Gemäß einer weiteren besonders bevorzugten Erfindungsvariante kann vorgesehen sein, dass die wenigstens eine Lufteinlassöffnung der Halte- und/oder Aufnahmeeinrichtung so ausgebildet ist, dass der wenigstens eine Luftstrom das wenigstens eine Speichermedium von unten her entlang einer definierten Wegstrecke umströmt und/oder so ausgebildet ist, dass diese mit wenigstens einem halte- und/oder aufnahmeeinrichtungsseitigen Strömungskanal strömungsverbunden ist, mittels dem der wenigstens eine Luftstrom zum Aufnahmeraum geführt ist. Diese Erfindungsvariante kann grundsätzlich alternativ zu den zuvor beschriebenen seitlichen Lufteinlassöffnungen der Halte- und/oder Aufnahmeeinrichtung vorgesehen sein, die den freiliegenden, das heißt nicht-überdeckten Luftauslassöffnungen der Andockstation zugeordnet sind. Besonders bevorzugt ist diese Erfindungsvariante jedoch zusätzlich zu der wenigstens einen halte- und/oder aufnahmeeinrichtungsseitig ausgebildeten seitlichen Lufteinlassöffnung, der wenigstens eine freiliegende und nicht von der Halte- und/oder Aufnahmeeinrichtung überdeckte andockstationsseitige Luftauslassöffnung zugeordnet ist, vorgesehen. Mit einem derartigen Aufbau lässt sich eine besonders effektive Substanzanreicherung der Strömungsluft erzielen, da zum Beispiel in Verbindung mit einem Membranbereich eines Speichermediums sichergestellt ist, das eine hohe Luftmenge über bzw. entlang dieses Membranbereichs strömt und eine sehr hohe Substanzabgabe bzw. Substanzdiffusion in die Umgebung bewirkt wird.

Um aus der Halte- und/oder Aufnahmeeinrichtung ausströmen zu können, ist an dieser wenigstens eine Abströmöffnung vorgesehen. Diese kann zum Beispiel durch wenigstens eine Zugangsöffnung des Aufnahmeraums ausgebildet sein, über die das wenigstens eine Speichermedium in den Aufnahmeraum einsetzbar ist. Alternativ oder zusätzlich kann diese wenigstens eine Abströmöffnung in einer Doppelfunktion aber auch durch die das Einströmen des wenigstens einen Luftstroms in den Aufnahmeraum ermöglichende, wenigstens eine seitliche Lufteinlassöffnung gebildet sein.

Der Membranbereich eines Speichermediums, über den die abzugebende bzw. zu verflüchtende Substanz vom Speichermedium abgegeben wird und welcher Membranbereich von dem wenigstens einen Luftstrom angeströmt und/oder umströmt wird, kann auf unterschiedliche Art und Weise bzw. durch unterschiedliche Materialien gebildet sein, so zum Beispiel durch eine Diffusionsfolie. Auch Fasermaterialen oder Textilmaterialen können zum Einsatz kommen.

Ein Aufbau des Speichermediums gemäß der Erfindung sieht vor, dass das Speichermedium durch ein Behältnis gebildet ist, in dem die wenigstens eine abzugebende bzw. zu verflüchtigende Substanz, zum Beispiel in Form einer Flüssigkeit und/oder eines Gels, aufgenommen ist, wobei ein definierter Behältniswandbereich als Membranbereich ausgebildet ist. Dieser Membranbereich ist bevorzugt mit einer abnehmbaren Abdeckung, zum Beispiel mit einer Abziehfolie oder dergleichen abgedeckt und/oder abgedichtet. Diese Abziehfolie kann zum Beispiel aus einem Aluminiummaterial oder dergleichen ausgebildet sein. Diese Abdeckung wird unmittelbar vor dem Einsetzen des Speichermediums in die Vorrichtung entfernt, so dass die Substanzabgabe erst in gewünschtem Maße in der Vorrichtung selbst erfolgt und nicht bereits vorher, wenn das Speichermedium noch nicht in der gewünschten Weise im Einsatz ist.

Das wenigstens eine Speichermedium weist erfindungsgemäß eine plättchenförmige und/oder rechteckige Form auf, wobei bevorzugt ein Membranbereich des Speichermediums wenigstens bereichsweise durch wenigstens eine der beiden großflächigen Seitenflächen gebildet ist. Dadurch wird eine große Fläche zur Verfügung gestellt, über die die Substanz aus dem Behälter in die Umgebung diffundieren kann. Des Weiteren wird eine große Angriffsfläche für die Luftströmung zur Verfügung gestellt, was die Effektivität der Anreicherung der Umgebungsluft mit der gewünschten Substanz erhöht.

Ein derartiges plättchenförmiges und/oder rechteckförmiges Speichermedium wird erfindungsgemäß über eine halte- und/oder aufnahmeraumseitige Zugangsöffnung in die Halte- und/oder Aufnahmeeinrichtung eingesetzt, bevorzugt mittels einer Art Führungskulissenanordnung geführt eingesteckt und/oder positioniert. Der Halte- und/oder Aufnahmeraum weist wenigstens einen Einsteckschlitz mit beabstandet gegenüberliegenden Führungsnuten auf, in denen das plättchenförmige und/oder rechteckförmige Speichermedium mit randseitig gegenüberliegenden Randplattenbereichen eingreift und geführt in die vorgegebene Speichermediumposition überführbar und/oder dort abstützbar und/oder haltbar ist. Eine derartige Ausgestaltung des Aufnahmeraums der Halte- und/oder Aufnahmeeinrichtung mit wenigstens einem Einsteckschlitz ergibt eine besonders einfache und funktionssichere Bedienung durch den Bediener beim Einsetzen oder Austauschen oder Nachfüllen eines Speichermediums.

Grundsätzlich kann die Halte- und/oder Aufnahmeeinrichtung bzw. deren Aufnahmeraum so ausgebildet sein, dass in diese bzw. diesen lediglich ein einziges Speichermedium eingesetzt werden kann. Bevorzugt ist jedoch ein Aufnahmeraum, in den mehrere, insbesondere zwei im Wesentlichen gleich ausgebildete und/oder voneinander quer zur Einsteckrichtung beabstandete Einsteckschlitze vorgesehen sind. Dadurch können gleiche oder unterschiedliche Substanzen verflüchtigt werden, zum Beispiel zwei unterschiedliche und miteinander harmonisierende Duftstoffe oder aber auch ein Duftstoff und ein Insektizid oder aber auch zwei Insektizide, um nur einige Beispiele zu nennen. Selbstverständlich ist auch mit einem derartigen Aufbau auch lediglich der Einsatz eines einzigen Speichermediums möglich. Die restlichen Einsteckschlitze sind dann in diesem Fall nicht mit Speichermedien bestückt.

Für ein funktionssicheres Positionieren ist es des Weiteren vorteilhaft, wenn das in den Einsteckschlitz eingesteckte Speichermedium mittels eines halte- und/oder aufnahmeeinrichtungsseitigen und/oder andockstationsseitigen Anschlagelementes in der definierten Einsteckposition abgestützt ist. Hierzu sind beispielsweise im Aufnahmeraum der Halte- und/oder Aufnahmeeinrichtung, zum Beispiel am der Einstecköffnung gegenüberliegenden Einsteckschlitzende, entsprechende Anschlagnasen vorgesehen.

Das bevorzugt durch eine Ventilatoranordnung gebildete wenigstens eine Mittel zum Erzeugen wenigstens eines Luftstroms wird vorzugsweise elektrisch betätigt, und zwar vorzugsweise mittels wenigstens eines Energiespeichers, zum Beispiel einer Batterie oder eines Akkumulators. Alternativ dazu kann die Energieversorgung aber selbstverständlich auch über ein elektrisches Stromnetz erfolgen. Im letzteren Falle führt dann von der Vorrichtung, zum Beispiel von der Andockstation ein Kabel weg, das mittels einer Steckdose verbindbar ist. Gegebenenfalls kann die Steckereinheit aber auch direkt an der Vorrichtung, zum Beispiel an der Andockstation angebracht sein, so dass in diesem Fall auf ein Kabel verzichtet werden kann. Um den Energiespeicher an der Vorrichtung unterzubringen, weist diese vorzugsweise ein Aufnahmefach für den Energiespeicher auf, welches Aufnahmefach bevorzugt mittels wenigstens eines Abdeckelements, zum Beispiel einer Klappe oder eines Deckels verschließbar ist. Dieses Abdeckelement kann entweder schwenkbar angelenkt oder aber auch vollständig abnehmbar ausgebildet sein.

Für einen besonders kompakten Aufbau ist vorgesehen, dass bei einer Mehrzahl von Energiespeichern oder, im Falle von in Aufnahmefächern aufgenommenen Energiespeichern, deren Aufnahmefächer zwischen sich einen Montagefreiraum ausbilden, in dem die wenigstens eine Ventilatoranordnung, gegebenenfalls zusammen mit weiteren Bauteilen der Vorrichtung aufgenommen und/oder montiert ist. Die weiteren Bauteile können dabei zum Beispiel weitere elektrische Komponenten, wie zum Beispiel eine Leiterplatte und/oder ein Chip und/oder eine Leuchteinrichtung oder dergleichen sein.

Beispielsweise ist in Verbindung mit dem wenigstens einen Energiespeicher vorgesehen, dass dieser, bezogen auf die Betriebsposition der Vorrichtung, horizontal und/oder vertikal im Inneren der Andockstation ausgerichtet ist. Bei einer im Wesentlichen horizontalen Ausrichtung der Energiespeicher ergibt sich ein insgesamt flacher, mehr scheibenförmiger Aufbau der Andockstation, von der dann zum Beispiel eine zuvor beschriebene Halte- und/oder Aufnahmeeinrichtung für das wenigstens eine Speichermedium bei auf einer Aufstandsfläche abgestellter Andockstation im Wesentlichen nach oben abragt, insbesondere turmartig abragt.

Im Falle von vertikal ausgerichteten Energiespeichern und/oder Aufnahmefächern kann am Gehäuse der Andockstation eine gabelartige, insbesondere U-förmige Aufnahme ausgebildet werden, die das wenigstens eine Speichermedium bzw. die dieses gegebenenfalls aufnehmende Halte- und/oder Aufnahmeeinrichtung wenigstens bereichsweise umgreift und/oder abstützt, insbesondere wenigstens bereichsweise formschlüssig und/oder in einer Anlageverbindung umgreift und/oder abstützt.

Grundsätzlich besteht die Möglichkeit, dass das erfindungsgemäße Gerät lediglich eine einzige Betriebsart bzw. lediglich einen einzigen Betriebsmodus anbietet, zum Beispiel dergestalt, dass bei aktivierter Vorrichtung eine bestimmte Luftmenge von der wenigstens einen Ventilatoranordnung erzeugt wird und diese zum Beispiel über die andockstationsseitigen Luftauslassöffnungen zu den zum Beispiel seitlichen aufnahmeeinrichtungsseitigen Lufteinlassöffnungen strömt, ohne dass die Luftmenge, die Luftgeschwindigkeit bzw. die Einström- und Ausströmquerschnitte der Lufteinlass- und Luftauslassöffnungen veränderbar sind. Alternativ hierzu kann jedoch aber auch wenigstens eine Steuer- und/oder Regeleinrichtung vorgesehen sein, mittels der die wenigstens eine Ventilatoranordnung in Abhängigkeit von definierten Betriebsparametern angesteuert wird, insbesondere zur Einstellung der Geschwindigkeit und/oder der Menge des zum wenigstens einen Speichermedium geführten wenigstens einen Luftstroms. Beispielsweise kann mittels der Steuer-und/oder Regeleinrichtung die wenigstens eine Ventilatoranordnung so angesteuert werden, dass zum Beispiel die Drehzahl eines Lüfterrades verringert oder erhöht wird, um eine entsprechende Anpassung der Luftmenge durchzuführen bzw. für den gerade gewählten Betriebsmodus einzustellen. Alternativ oder zusätzlich dazu kann aber auch wenigstens ein Verschlusselement vorgesehen sein, zum Beispiel eine Klappe oder dergleichen, mittels der der Öffnungsquerschnitt der wenigstens einen Luftauslassöffnung in Abhängigkeit von vorgegebenen Betriebsparametern in einem definierten Maße gesperrt oder freigegeben wird. Ein derartiges Verschlusselement kann zum Beispiel manuell verstellbar ausgebildet sein. Grundsätzlich besteht aber auch hier eine Ansteuerungsmöglichkeit über eine zum Beispiel Steuer- und/oder Regeleinrichtung in Abhängigkeit von definierten Betriebsparametern, wie zum Beispiel die Geschwindigkeit und/oder die Menge des Luftstroms, wobei eine derartige Verstellung vorzugsweise mittels entsprechender Aktuatoren erfolgt.

Gemäß einer weiteren besonders bevorzugten Ausgestaltung weist die Steuer-und/oder Regeleinrichtung eine Speichereinrichtung auf, in der wenigstens ein, vorzugsweise mehrere unterschiedliche vorprogrammierte Betriebsmodi und/oder Betriebszyklen abgelegt ist bzw. sind. Damit können je nach Bedienerwunsch unterschiedliche Programme gefahren werden und zum Beispiel unterschiedliche Substanzabgabemengen eingestellt werden, zum Beispiel tageszeitabhängig und/oder saisonabhängig und/oder anwendungsabhängig.

Besonders bevorzugt ist in Verbindung mit der erfindungsgemäßen Vorrichtung eine Verfahrensführung, bei der zum Beispiel in einem Grundmodus die wenigstens eine Ventilatoranordnung deaktiviert ist und die wenigstens eine abzugebende Substanz ohne Luftstromunterstützung von der Ventilatoranordnung aus dem Speichermedium abdampft bzw. von diesem abgegeben wird. Weiter ist dann wenigstens ein zusätzlicher Betriebsmodus vorgesehen, in dem die wenigstens eine Ventilatoranordnung dann für eine definierte Zeitdauer aktiviert sein kann und dementsprechend ein die Substanzabgabe unterstützender Luftstrom erzeugt werden kann. Auch hier, in Verbindung mit diesem wenigstens einen zusätzlichen Betriebsmodus, können wieder unterschiedliche Programmführungen bzw. Verfahrensführungen vorgesehen sein, so zum Beispiel ein Betriebsmodus, in dem die wenigstens eine Ventilatoranordnung dauerhaft aktiviert ist. Alternativ hierzu oder aber auch zusätzlich hierzu kann zum Beispiel wenigstens ein zusätzlicher Betriebsmodus vorgesehen sein, in dem die wenigstens eine Ventilatoranordnung für eine definierte Zeitdauer aktiviert und anschließend für eine definierte Zeitdauer deaktiviert ist. Hier ist dann bevorzugt weiter vorgesehen, dass sich die einzelnen Aktivitäts-und Deaktivitätsphasen periodisch bzw. zyklisch wiederholen. Besonders bevorzugt ist im Falle mehrerer zusätzlicher Betriebsmodi vorgesehen, dass sich diese hinsichtlich der Aktivitäts- und/oder Deaktivitätsphasen und/oder der Betriebsart, insbesondere der Drehzahl, der wenigstens einen Ventilatoranordnung unterscheiden. Beispielsweise kann zur Anwahl der unterschiedlichen Betriebsmodi ein einziges Schalterelement vorgesehen sein, mittels dem zum Beispiel in einer ersten Schalterstellung die wenigstens eine Ventilatoranordnung nicht betätigt ist, dagegen in einer zweiten Schalterstellung die Ventilatoranordnung für eine definierte Zeitdauer, zum Beispiel für 15 min. aktiviert und für eine weitere, darauffolgende Zeitdauer, zum Beispiel wiederum 15 min., deaktiviert ist. Diese Aktivitäts- und/oder Deaktivitätsphasen wiederholen sich dann programmgesteuert solange, bis das Schalterelement wieder in eine andere Position gebracht wird. Beispielsweise kann dann noch eine weitere Schalterelementstellung vorgesehen sein, in der die wenigstens eine Ventilatoranordnung beispielhaft für längere Zeit betätigt wird, zum Beispiel für 30 min., während die Deaktivitäsphase hier dann kürzer gewählt ist, zum Beispiel auch bei 15 min. liegen kann. Des Weiteren kann zum Beispiel auch noch eine weitere Schalterelementstellung vorgesehen sein, in der die Ventilatoranordnung zum Beispiel dauerhaft in Betrieb ist. Unabhängig von der Möglichkeit, hier über die Intensität der Substanzabgabe in die Umgebung einstellen zu können, besteht hier auch der weitere Vorteil insbesondere darin, dass zum Beispiel auch die Lebensdauer der Batterien geschont werden kann, indem Anwähl- und Betriebsmöglichkeiten zur Verfügung gestellt werden, in denen die wenigstens eine Ventilatoranordnung dauerhaft bzw. für eine definierte Zeitspanne nicht betätigt wird. Ganz allgemein gesehen, ist somit gemäß einer besonders bevorzugten Ausgestaltung der vorliegenden Erfindungsidee vorgesehen, dass die Vorrichtung wenigstens ein, vorzugsweise von außen her zugängliches Schaltelement aufweist, mittels dem oder den der jeweils gewünschte Betriebszustand und/oder Betriebsmodus auswählbar und/oder die Vorrichtung, insbesondere die wenigstens eine Ventilatoranordnung betätigbar und/oder ein- oder ausschaltbar ist. Hierzu können grundsätzlich mehrere Schalter vorgesehen sein, zum Beispiel ein erster Schalter, um die Vorrichtung ein- bzw. auszuschalten und ein zweiter Schalter, um zum Beispiel den jeweils gewünschten Betriebszustand und/oder Betriebsmodus auszuwählen. Insbesondere hier kann dann zum Beispiel vorgesehen sein, dass die Betriebsmodi keinen Betriebsmodus aufweisen, bei dem die Ventilatoranordnung abgeschaltet ist, da dieser Betriebszustand durch das mit dem ersten Schalter bewirkte Ausschalten der Vorrichtung bereits erreicht ist. Grundsätzlich kann aber auch ein einziger Schalter vorgesehen sein, mittels dem die Vorrichtung sowohl ein- bzw. ausschaltbar und auch der jeweils gewünschte Betriebszustand und/oder Betriebsmodus auswählbar ist, wie bereits zuvor erläutert.

Weiter kann wenigstens eine Leuchteinrichtung vorgesehen sein, zum Beispiel wenigstens eine Leuchtdiode oder dergleichen, mittels der der jeweilige Betriebszustand und/oder Betriebsmodus anzeigbar und/oder die Vorrichtung, insbesondere die Andockstation und/oder das wenigstens eine Speichermedium und/oder wenigstens ein das wenigstens eine Speichermedium aufnehmende Bauteil beleuchtbar und/oder ausleuchtbar ist. Dies ist besonders dann vorteilhaft, wenn die Halte- und/oder Aufnahmeeinrichtung, die das wenigstens eine Speichermedium aufnimmt, wenigstens bereichsweise aus einem transparenten Material ausgebildet ist, zum Beispiel aus einem Glas- und/oder Kunststoffmaterial, da dann in bestimmten, definierten Bereichen der Halte- und/oder Aufnahmeeinrichtung gleichzeitig auch ein bestimmter Lichteffekt erzeugt werden kann. Insbesondere im letzteren Falle kann dabei vorgesehen sein, dass die wenigstens eine Leuchteinrichtung im angedockten Zustand von der Halte- und/oder Aufnahmeeinrichtung überdeckt ist.

Wie bereits eingangs erläutert, ist gemäß einer besonders vorteilhaften erfindungsgemäßen Lösung die Vorrichtung als Set ausgebildet mit mehreren unterschiedlichen Halte- und/oder Aufnahmeeinrichtungen, die mit der Andockstation kompatibel sind, so dass bevorzugt lediglich eine einzige Andockstation Bestandteil dieses Sets ist, das dann mit unterschiedlichen Halte- und/oder Aufnahmeeinrichtungen individuell bestückt und ausgestaltet werden kann. Selbstverständlich kann auch wenigstens ein Speichermedium Setbestandteil sein.

Auch eine Verfahrensführung, zum Beispiel in Verbindung mit der Auswahlmöglichkeit unterschiedlicher Betriebsmodi wird ganz allgemein beansprucht.

Die Erfindung wird nachfolgend anhand einer Figur näher erläutert.

Es zeigen:
- Fig. 1: schematisch unterschiedliche Ansichten einer ersten Ausführungsform der vorliegenden Erfindungsidee,
- Fig. 2: schematisch eine zweite Ausführungsform der vorliegenden Erfindungsidee,
- Fig. 3: schematisch eine dritte Ausführungsform der vorliegenden Erfindungsidee,
- Fig. 4: schematisch unterschiedliche Ansichten einer vierten Ausführungsform der vorliegenden Erfindungsidee,
- Fig. 5: schematisch eine bevorzugte Ausgestaltung eines erfindungsgemäßen Speichermediums,
- Fig. 6: verschiedene Detailansichten zu einer Aufnahmeeinrichtung.

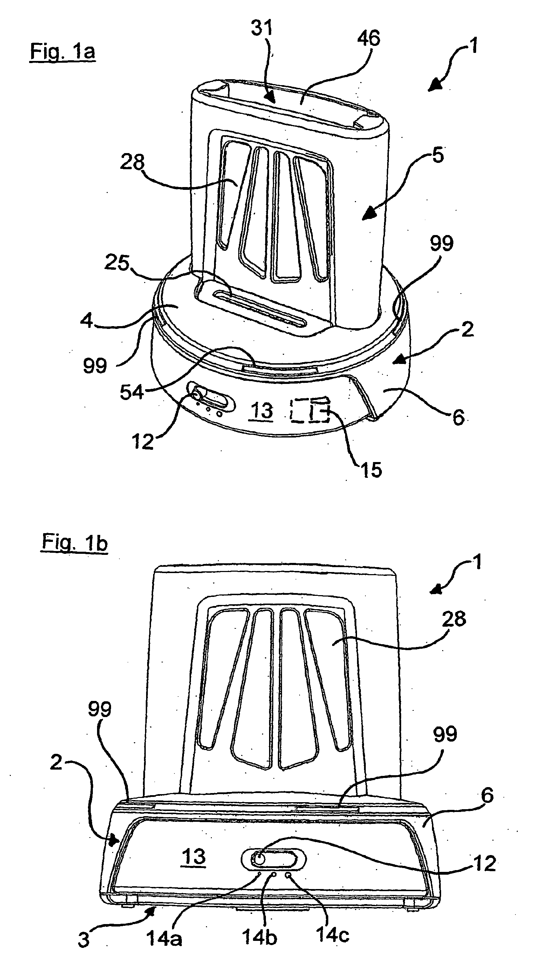

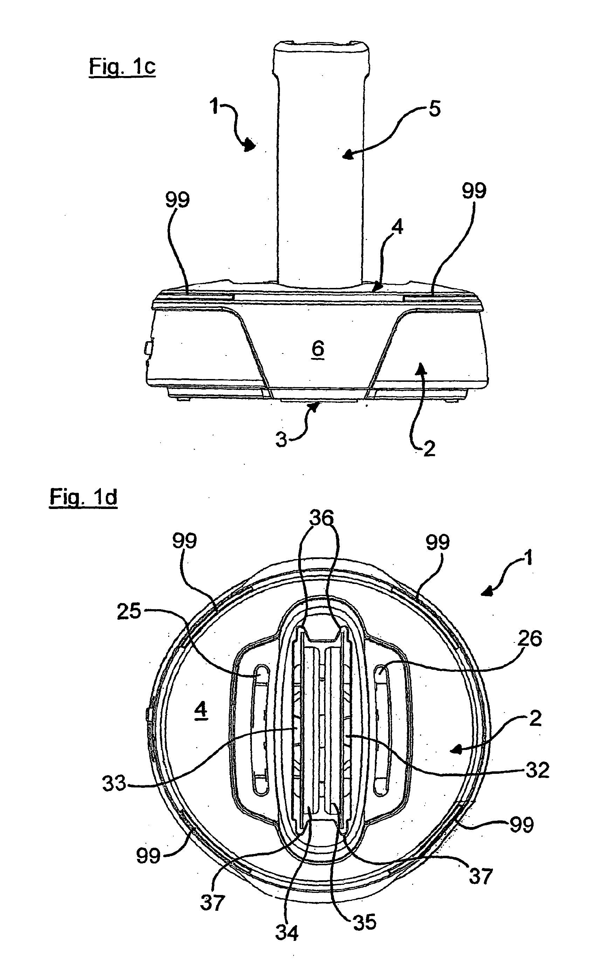

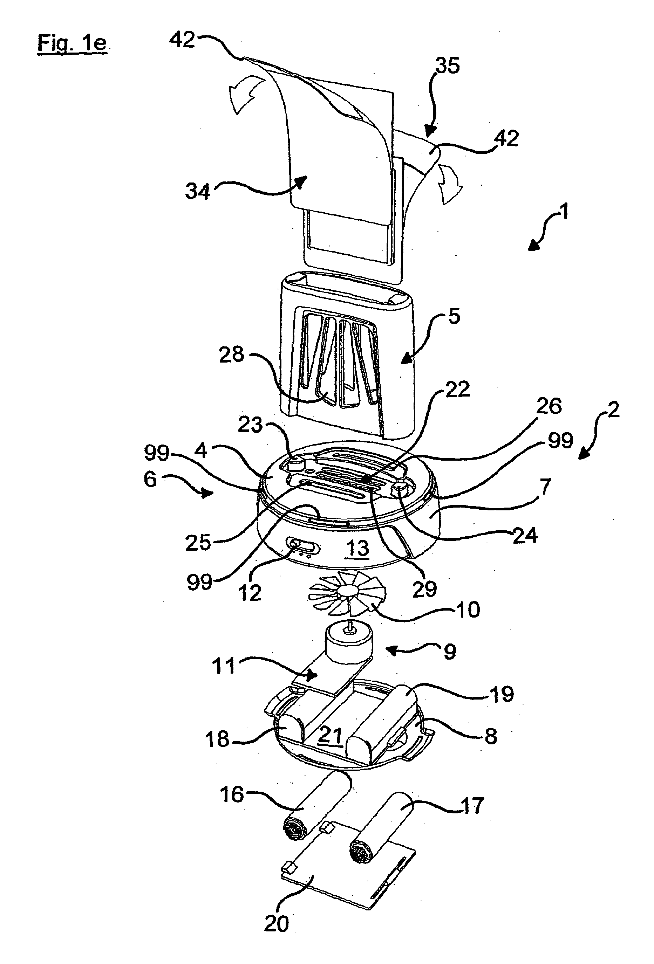

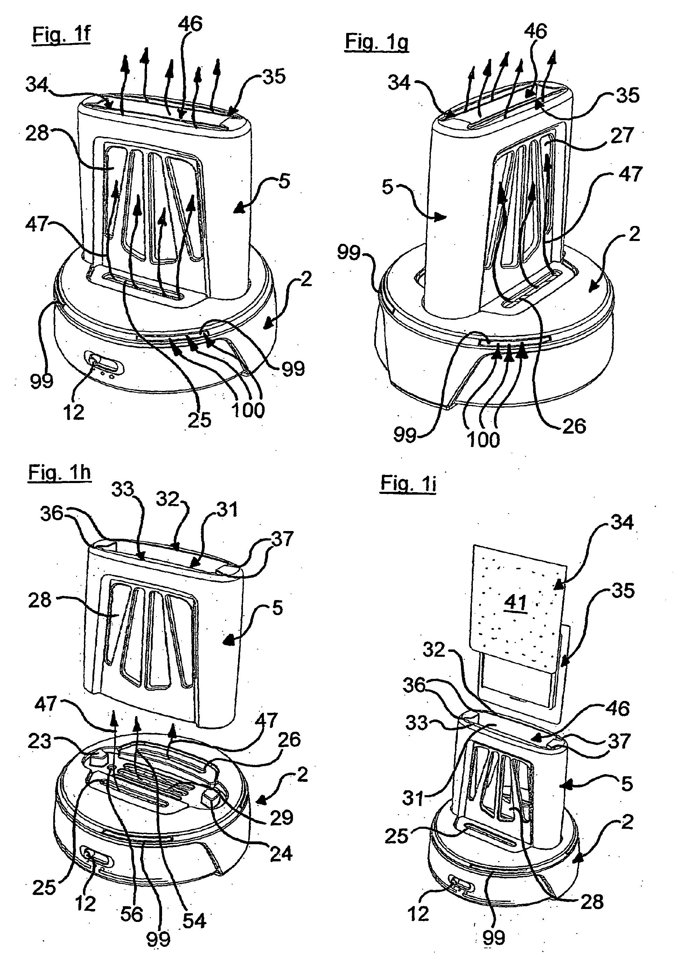

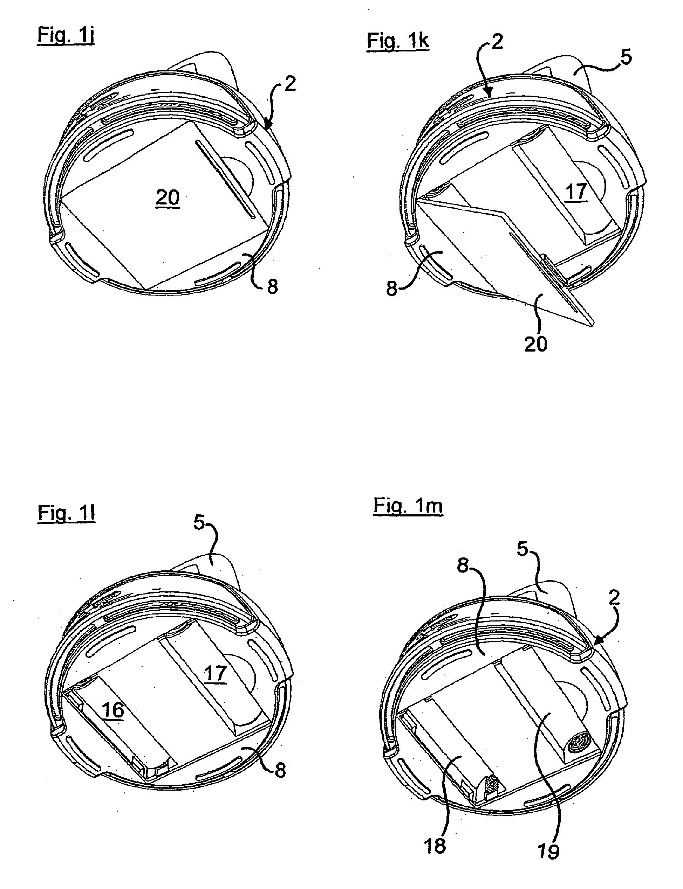

In der Fig. 1 sind unterschiedliche Ansichten einer ersten beispielhaften erfindungsgemäßen Ausführungsform gezeigt. So zeigt die Fig. 1a eine schematische, perspektivische Vorderansicht, die Fig. 1b eine Vorderansicht, die Fig. 1 c eine Seitenansicht und die Fig. 1d eine Draufsicht auf die Vorrichtung 1 im zusammengebauten Zustand. Diese Vorrichtung 1 weist als Basisteil eine Andockstation 2 auf, die hier als Standgerät mit einer Aufstandsfläche 3 und einer Anschlussfläche 4 für eine hier lediglich Aufnahmeeinrichtung 5 bezeichnete Halte-und/oder Aufnahmeeinrichtung ausgebildet ist.

Die Andockstation 2 selbst ist durch ein hier beispielhaft eine in etwa kreisförmige Scheibengeometrie aufweisendes Gehäuse 6 gebildet, das, wie dies aus der perspektivischen Darstellung der Fig. 1e ersichtlich ist, wiederum durch ein Gehäuseoberteil 7 und ein Gehäuseunterteil 8 gebildet ist.

Das Gehäuseoberteil 7 und das Gehäuseunterteil 8 sind zum Beispiel durch eine hier nicht im Detail dargestellte Clip- und/oder Rastverbindung miteinander lösbar verbindbar, wobei diese entsprechend randseitig am Gehäuseoberteil 7 und am Gehäuseunterteil 8, einander zugeordnet, ausgebildet sein können. Im Inneren des Gehäuses 6 ist, wie dies insbesondere aus der Fig. 1b ersichtlich ist, eine elektrisch betätigbare Ventilatoranordnung 9 angeordnet, die ein Flügelrad 10 aufweist, um in noch näher zu beschreibender Weise einen Luftstrom zu erzeugen. Die Ventilatoranordnung 9 selbst ist mit einer Steuer- und/oder Regeleinrichtung 11, die in der Darstellung der Fig. 1b lediglich beispielhaft und schematisch durch eine Leiterplatte repräsentiert ist, gekoppelt, mittels der in Abhängigkeit von einer Stellung eines Handschalters 12 (siehe Fig. 1a, 1b) in einem Seitenwandbereich 13 des Gehäuses 6 der Andockstation 2 ein bestimmter Betriebsmodus ausgewählt werden kann. Im hier gezeigten Beispielfall können zum Beispiel drei unterschiedliche Betriebsmodi bzw. Betriebszustände angewählt werden, die durch unterschiedliche Markierungen 14a, 14b und 14c (Fig. 1b) gekennzeichnet sind und zwar dergestalt, dass bei einer Schalterpositionierung im Bereich der Markierung 14a die Ventilatoranordnung 9 zum Beispiel ausgeschaltet ist. In einer auf der Markierung 14b stehenden Handschalterstellung kann dann zum Beispiel die Ventilatoranordnung 9 mit einer definierten Drehzahl für eine definierte Zeit, zum Beispiel 15 min, aktiviert sein. Im Anschluss daran kann die Ventilatoranordnung 9 dann für eine definierte Zeit, zum Beispiel ebenfalls 15 min. deaktiviert sein. Nach dieser Deaktivierungsphase wird die Ventilatoranordnung 9 dann wieder aktiviert und der Zyklus beginnt von Neuem. Bei einer Handschalterstellung im Bereich der Markierung 14c ändert sich dann der Betriebsmodus wieder und wird zum Beispiel die Zeitdauer der Aktivierungs- und/oder Deaktivierungsphasen gegenüber der- bzw. denjenigen der Schalterstellung 14b verändert, zum Beispiel dergestalt, dass die jeweilige Aktivierungsphase länger dauert, zum Beispiel 30 min., während die Deaktivierungsphase zum Beispiel gleich bleibt, das heißt im Beispielfall bei 15 min.. Grundsätzlich könnte in der Schalterstellung 14c auch vorgesehen sein, dass die Ventilatoranordnung 9 dauerhaft aktiviert ist. Dies könnte gegebenenfalls auch mit einer zusätzlichen, hier nicht gezeigten Schalterstellung realisiert werden. Die vorherigen Ausführungen sollen lediglich beispielhaft die Variationsmöglichkeiten verdeutlichen.

Beispielsweise kann auch, wie dies in der Fig. 1a lediglich schematisch und strichliert eingezeichnet ist, ein zusätzlicher, separater Handschalter 15 vorgesehen sein, mittels dem die Vorrichtung 1 ein- bzw. ausschaltbar ist, so dass mit dem Handschalter 15 nur der jeweilige Betriebsmodus ausgewählt wird.

Wie dies insbesondere aus einer Zusammenschau der Fig. 1 e mit den Fig. 1j bis 1 m hervorgeht, sind zur elektrischen Betätigung der Ventilatoranordnung 9 Batterien 16, 17 vorgesehen, die in über die Aufstandsfläche 3 der Andockstation 2 zugängliche Aufnahmefächer 18, 19 in das Gehäuseunterteil 8 einsetzbar sind. Hierzu ist an der Aufstandsfläche 3 des Gehäuseunterteils 8 und damit des Gehäuses 6 ein abnehmbarer sowie lösbar verrastbarer Deckel 20 vorgesehen, der im abgenommenen Zustand des Deckels 20 die Aufnahmefächer 18, 19 freigibt. In den Fig. 1j bis 1 m ist gezeigt, wie der Deckel 20 entfernt und die Batterien 16, 17 aus den Ausnahmefächern 18, 19 entfernt werden können.

Wie dies insbesondere aus der Fig. 1e hervorgeht, stehen die Aufnahmefächer 18, 19 von der Oberseite des Gehäuseunterteils 8 ab und bilden auf gegenüberliegenden Seiten des Gehäuseunterteils 8 horizontal liegende, vorsprungartige Erhebungen aus, die zwischen sich einen Montagefreiraum 21 ausbilden, in dem im montierten Zustand die Ventilatoranordnung 9 gegebenenfalls mitsamt den weiteren elektronischen Bauteilen einer Steuer- und/oder Regeleinrichtung 11 aufgenommen sind. Dadurch ergibt sich eine insgesamt flache und kompakte Bauweise des Gehäuses 6.

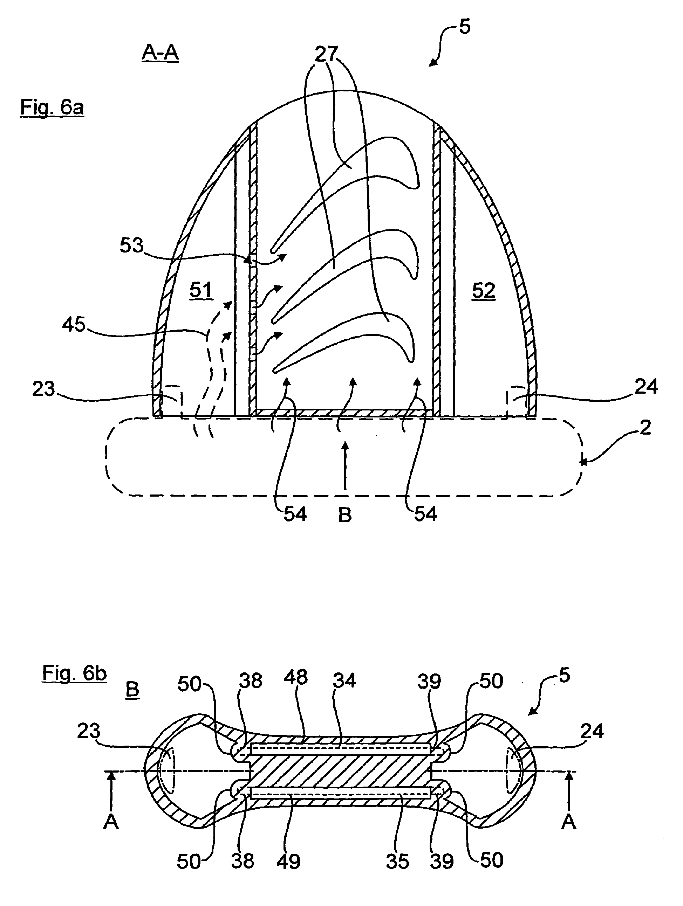

Wie bereits vorbeschrieben, weist das Gehäuseoberteil 7 eine Anschlussfläche 4 für die Aufnahmeeinrichtung 5 auf, die in einem Andockungsbereich 22 angedockt wird. In diesem Andockungsbereich 22 sind, wie dies insbesondere aus der Fig. 1e ersichtlich ist, zwei beabstandete und in etwa gegenüberliegende Rastvorsprünge 23, 24 vorgesehen, die Rastelemente ausbilden und die, wie dies insbesondere auch aus der Fig. 6a ersichtlich ist, so in die Aufnahmeeinrichtung 5 eingreifen, dass die Aufnahmeeinrichtung mit einer vorgegebenen Haltekraft lösbar an der Andockstation 2 gehalten und/oder abgestützt ist.

Im angedockten Zustand der Aufnahmeeinrichtung 5, wie dies zum Beispiel in den Fig. 1f, 1g und 1i dargestellt ist, liegt auf gegenüberliegenden Seiten der Längsseiten der Aufnahmeeinrichtung 5 jeweils ein Luftauslassschlitz 25, 26, dem im Bereich der Aufnahmeeinrichtung jeweils seitliche Lufteinlassschlitze 27, 28 zugeordnet sind.

Hier, wie auch bei allen nachfolgenden Ausführungsbeispielen ist die Anzahl und/oder die Form der jeweils vorgesehenen Lufteinlass- und/oder Luftauslassöffnungen lediglich beispielhaft gewählt. Selbstverständlich sind auch andere Formen bezüglich der zum Beispiel Schlitzgeometrie oder eine andere Anzahl der Öffnungen bzw. Schlitze jederzeit denkbar.

Ferner ist hier weiter vorgesehen, dass im Bereich zwischen den beiden freiliegenden Luftauslassschlitzen 25, 26, das heißt im Bereich zwischen den beiden gegenüberliegenden Rastvorsprüngen 23, 24 hier beispielhaft drei weitere innenliegende Luftaustassschlitze 29 angeordnet sind, die im angedockten Zustand der Aufnahmeeinrichtung 5 von der Aufnahmeeinrichtung 5 abgedeckt sind.

Ferner sind am Gehäuse 6 auch noch ein oder mehrere Lufteinlassschlitze 99 vorgesehen, über die, wie dies insbesondere aus der Fig. 1f und Fig. 1e ersichtlich ist, Umgebungsluft 100 in das Gehäuseinnere eingesaugt wird. Die Lufteinlassschlitze 99 sind im vorliegenden Beispielfall voneinander beabstandet im oberen Eckbereich des Gehäuses 6 ausgebildet, können grundsätzlich aber auch an anderen Stellen angeordnet sein, zum Beispiel im Seitenwandbereich 13 des Gehäuseoberteils 7.

Die Ventilatoranordnung 9 mitsamt Lüfterrad 10 ist hier zudem so im Gehäuse 6 aufgenommen und angeordnet, dass mittels dieser der Luftstrom 47 unmittelbar auf die Luftauslassschlitze 25, 26 bzw. 29 gerichtet ist.

Wie dies aus den Fig. 1 weiter ersichtlich ist, weist die Aufnahmeeinrichtung weiter einen Aufnahmeraum 31 mit zwei Einsteckschlitzen 32, 33 auf, in die jeweils ein plättchenförmig und rechteckförmig ausgebildetes Behältnis 34, 35 als Speichermedium für eine zu verflüchtigende Substanz einsteckbar ist.

Die beiden Einsteckschlitze 32, 33 sind quer zur Einsteckrichtung voneinander beabstandet und weisen jeweils gegenüberliegende Führungsnuten 36, 37 auf, in denen das plättchen- und rechteckförmige Behältnis mit randseitig gegenüberliegenden Randplattenbereichen 38, 39 eingreift und geführt in die zum Beispiel in der Fig. 1d in der Draufsicht gezeigte Endposition überführbar ist.

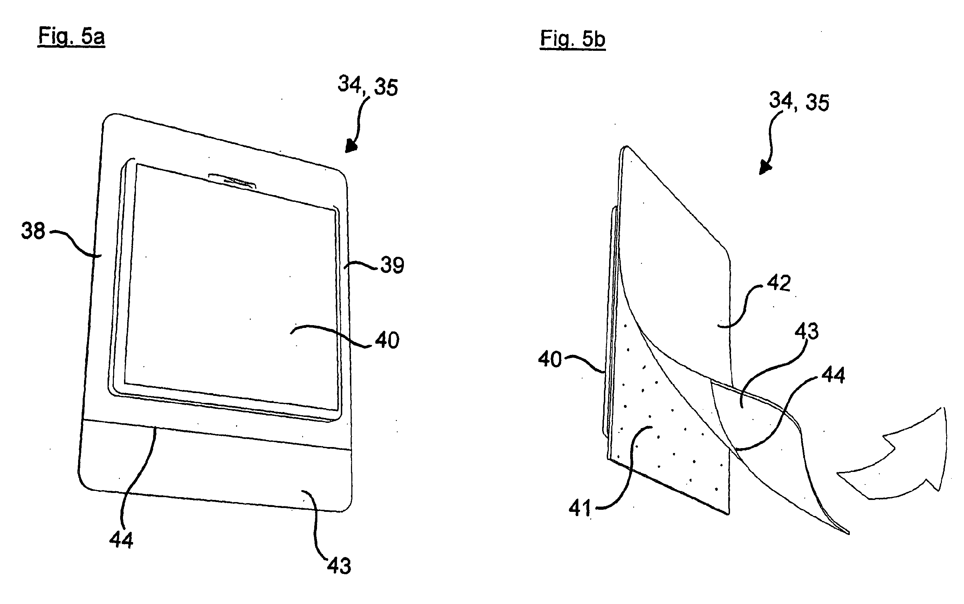

Wie dies insbesondere aus der Fig. 5a ersichtlich ist, weist das Behältnis eine wannenförmige Aufnahme 40 für ein zu verflüchtigendes Material, zum Beispiel einen Duftstoff und/oder ein Insektizid auf, das zum Beispiel durch ein Flüssigkeit oder ein Gel gebildet sein kann. Wie dies aus der Fig. 5a sehr gut ersichtlich ist, weist diese wannenförmige Aufnahme eine flache, quaderartige Außenkontur auf. Die wannenförmige Aufnahme 40 ist, wie dies insbesondere aus der Fig. 5b hervorgeht, mittels einer Membranfolie 41 abgedeckt, welche in der Fig. 5b mit einem Punktmuster versehen ist. Über diese einen Membranbereich ausbildende Membranfolie 41 kann die in der wannenförmigen Aufnahme 40 aufgenommene Flüssigkeit oder das dort aufgenommene Gel in die Umgebung ausdiffundieren. Damit dies erst dann geschieht, wenn das Behältnis 34 bzw. 35 in die Aufnahmeeinrichtung 5 eingesteckt wird, ist die Membranfolie 41 mittels einer Abdeckfolie 42 versiegelt bzw. abgedichtet, die, wie dies zum Beispiel in der Fig. 5b und auch in der Fig. 1 e dargestellt ist, unmittelbar vor dem Einsetzen der Behältnisse 34, 35 an einem Überstand 43 erfasst wird, gegebenenfalls entlang eines Knickbereichs 44 umgeknickt wird und anschließend von der Membranfolie 41 abgezogen wird.

Die Behältnisse 34, 35 werden dann so in die jeweils zugeordneten Einsteckschlitze 32, 33 eingesetzt, dass die Membranfolie 41 den seitlichen Lufteinlassschlitzen 27, 28 zugewandt ist. Wird nun die Vorrichtung 1 betätigt und die Ventilatoranordnung 9 mit einer definierten Drehzahl angetrieben, so wird Umgebungsluft 100 über die Lufteinlassschlitze 99 in das Gehäuse eingesaugt, anschließend im Gehäuse 6 der Andockstation 2 ein Luftstrom 47 erzeugt, der, wie dies insbesondere den Fig. 1f und 1g zu entnehmen ist, über die freiliegenden Luftauslassschlitze 25, 26 austritt und über die seitlichen Lufteinlassschlitze 27, 28 der Aufnahmeeinrichtung 5 in den Aufnahmeraum 31 der Aufnahmeeinrichtung 5 einströmt. Dort strömt dieser Luftstrom 47 dann entlang der Oberfläche der Membranfolie 41 der Behältnisse 34, 35 nach oben, wobei sich der Luftstrom 47 mit der durch die Membranfolie 41 hindurchdiffundierenden Substanz anreichert. Dieser mit der zu verflüchtigenden bzw. abzugebenden Substanz angereicherte Luftstrom strömt dann, wie dies in den Fig. 1f und 1g lediglich äußerst schematisch dargestellt ist, über eine Zugangsöffnung 46 des Aufnahmeraums 31 in die Umgebung ab.

Durch den über die Membranfolie 41 geführten Luftstrom 47 wird dabei die Abgaberate und/oder Abgabemenge der Substanz an die Umgebung erhöht, so dass die Effektivität der Vorrichtung im Hinblick auf zum Beispiel eine Wirkstoffabgabe wesentlich erhöht werden kann. Durch die grundsätzlich gegebene Möglichkeit der Drehzahlvariation und/oder die Anwahl unterschiedlicher Betriebsmodi oder Betriebszyklen könnte zudem die Geschwindigkeit des Luftstroms und auch die Menge des Luftstroms und damit die Diffusionsgeschwindigkeit der Substanz durch die Membranfolie 41 hindurch beeinflusst werden.

Die innenliegenden Luftauslassschlitze 29 können optional vorgesehen sein. Für den Fall, dass Sie, wie im hier vorliegenden Ausführungsbeispiel vorgesehen sind, kann über diese ein weiterer Luftstrom 54 in den Aufnahmeraum 31 der Aufnahmeeinrichtung 5 einströmen und somit auch von unten her eine zusätzliche Luftzufuhr zu den jeweiligen Membranfolien 41 der Behältnisse 34, 35 erzielt werden. Um diese Luftzufuhr zu ermöglichen, weist die Aufnahmeeinrichtung 5, zum Beispiel wie dies lediglich schematisch in der Fig. 6b dargestellt ist, an der Unterseite aufnahmeeinrichtungsseitige Lufteinlassschlitze 48, 49 auf.

In der Fig. 6b ist des Weiteren dargestellt, dass die hier lediglich beispielhaft zwei Lufteinlassschlitze 48, 49 lediglich eine solche Längserstreckung aufweisen, dass an den gegenüberliegenden Schlitzenden Anschläge 50 ausgebildet werden, an denen sich die Behältnisse 34, 35 mit ihren jeweiligen Randplattenbereichen 38, 39 in der eingesetzten Position abstützen. Die Behältnisse 34, 35 sind hier aus Übersichtlichkeitsgründen lediglich strichliert dargestellt.

Wie dies insbesondere in der Fig. 6a schematisch und strichliert dargestellt ist, können die im Andockbereich 22 vorgesehenen innenliegenden Luftauslassschlitze 29, 30 eine solche Länge aufweisen, dass diese in auf gegenüberliegenden Seiten der Aufnahmeeinrichtung 5 liegende Strömungskanäle 51, 52 einströmt und von dort über wenigstens eine innenliegende, seitliche Durchströmöffnung 53 in den Bereich der Behältnisse 34, 35, insbesondere in den Bereich der Membranfolien 41 einströmen oder überströmen kann. Dies ist in der Fig. 6a lediglich auf der linken Bildhälfte eingezeichnet, in Verbindung mit einem Luftstrom 45.

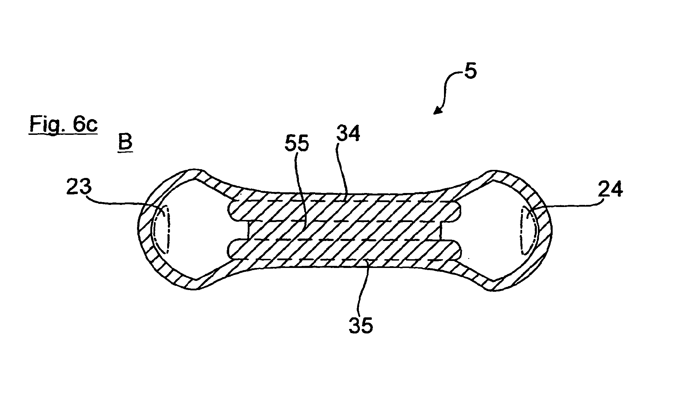

Für den Fall, dass innenliegende Luftauslassschlitze 29 vorliegen sollten und keine Einströmung in den Aufnahmeraum 31 der Aufnahmeeinrichtung 5 von unten her gewünscht sein sollte, kann die Aufnahmeeinrichtung auch ausgebildet sein, wie in der Fig. 6c dargestellt, nämlich mit einer bodenseitig, bevorzugt materialeinheitlich und einstückig mit der Aufnahmeeinrichtung 5 ausgebildeten Abdeckplatte 55, die ein Einströmen in den Aufnahmeraum 31 verhindert. In diesem Fall bildet dann die Abdeckplatte 55 gleichzeitig das Anschlagelement aus, das die in die Einsteckschlitze 31, 32 eingesteckten Behältnisse 34, 35 in der gewünschten Position abstützt.

Lediglich der Vollständigkeit halber sei noch erwähnt, dass die Fig. 6a hier einen Schnitt entlang der Linie A-A der Fig. 6b darstellt, während die Fig. 6b und 6c unterschiedliche alternative Ausgestaltungen der Aufnahmeeinrichtung 5 in Richtung des Pfeils B der Fig. 6a zeigen. Selbstverständlich lassen sich diese in der Fig. 6 gezeigten Ausgestaltungen auf sämtliche Ausführungsformen von Aufnahmeeinrichtungen dieser Erfindungsidee übertragen.

Wie dies insbesondere aus der Fig. 1e und 1h ersichtlich ist, ist im Bereich der Anschlussfläche 4 bzw. im von der Aufnahmeeinrichtung 5 überdeckten Andockbereich 22 eine Leuchtdiode 56 angeordnet, die zum Beispiel den Betriebszustand (EIN/AUS) der Vorrichtung 1 bzw. der Ventilatoranordnung 9 anzeigen kann. Für den Fall, dass die Aufnahmeeinrichtung zum Beispiel aus einem transparenten Kunststoff- und/oder Glasmaterial hergestellt ist, kann mittels einer derartigen Leuchtdiode 56, von der hier lediglich eine beispielhaft dargestellt ist, aber auch alternativ oder zusätzlich ein vorteilhafter Lichteffekt durch zum Beispiel Ausleuchtung der Aufnahmeeinrichtung 5 bzw. Ausleuchtung des Aufnahmeraums 31 bzw. Ausleuchtung der Behältnisse 34, 35 erzielt werden.

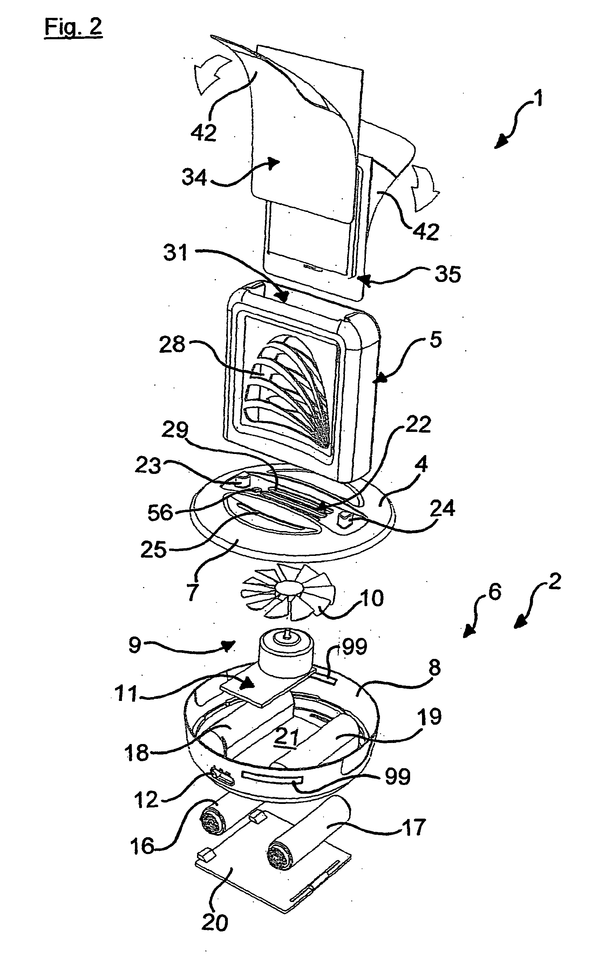

In der Fig. 2 ist eine alternative Ausführungsform gezeigt, die sich von derjenigen der Fig. 1 lediglich insofern unterscheidet, dass die Aufnahmeeinrichtung 5 ein anderes Muster der seitlichen Lufteinlassschlitze 27, 28 aufweist. Des Weiteren ist hier im Unterschied zur Ausführungsform gemäß der Fig. 1 das Gehäuseunterteil 8 wannen- bzw. schüsselförmig ausgebildet, während das Gehäuseoberteil 7 hier platten- bzw. scheibenförmig ausgebildet ist, das heißt, dass die Geometrie dieser beiden Gehäuseteile 7, 8 gegenüber derjenigen der Ausführungsform der Fig. 1 umgekehrt ist. Die gehäuseseitigen Lufteinlassschlitze 99 sind hier im Bereich der Seitenwand des Gehäuseunterteils 8 lediglich äußerst beispielhaft und schematisch dargestellt. Ansonsten entspricht der Aufbau demjenigen der Fig. 1, so dass diesbezüglich zur Vermeidung von unnötigen Wiederholungen auf die zuvor gemachten Ausführungen verwiesen wird, was insbesondere für die Funktionsweise der Vorrichtung gilt.

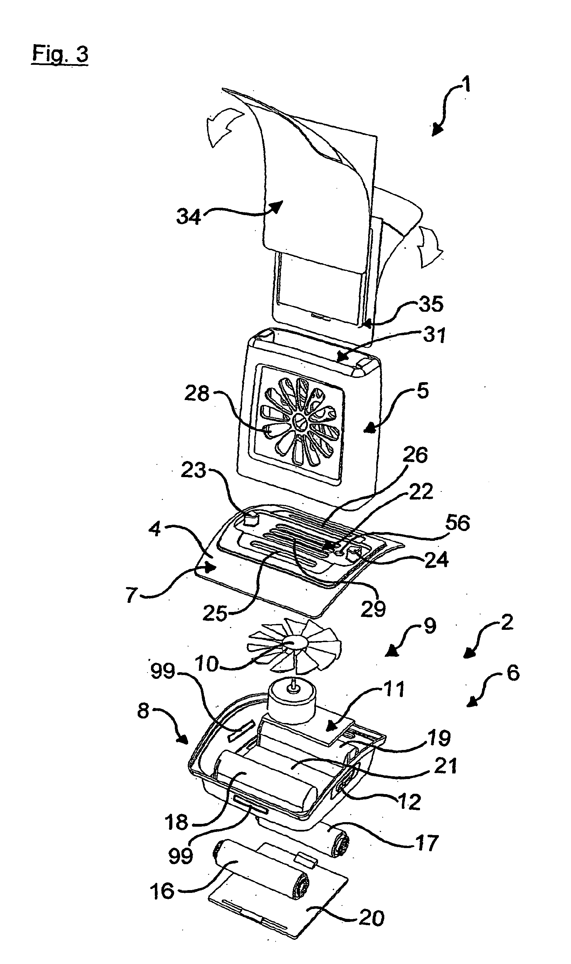

Das Gleiche trifft im Wesentlichen auf die Ausführungsvariante gemäß Fig. 3 zu, bei der die seitlichen Einlassschlitze 27, 28 der Aufnahmeeinrichtung 5 eine Art Blumenmuster ausbilden. Im weiteren Unterschied zur Ausgestaltung gemäß der Fig. 1 und gemäß der Fig. 2 ist hier die Andockstation 2 mit einem gewölbten, konvexen Gehäuseoberteil 7 ausgestaltet, das mit einem wannenförmigen Gehäuseunterteil 8 verbindbar ist. Ansonsten entspricht auch hier wiederum der Aufbau und die Funktionsweise demjenigen bzw. derjenigen, wie sie in Verbindung mit der Fig. 1 beschrieben worden ist.

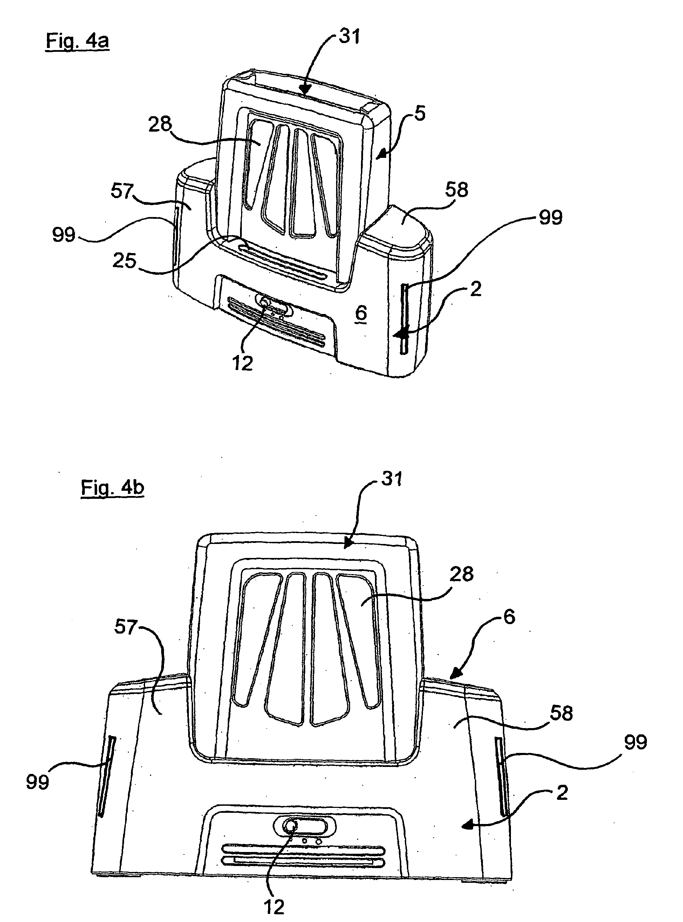

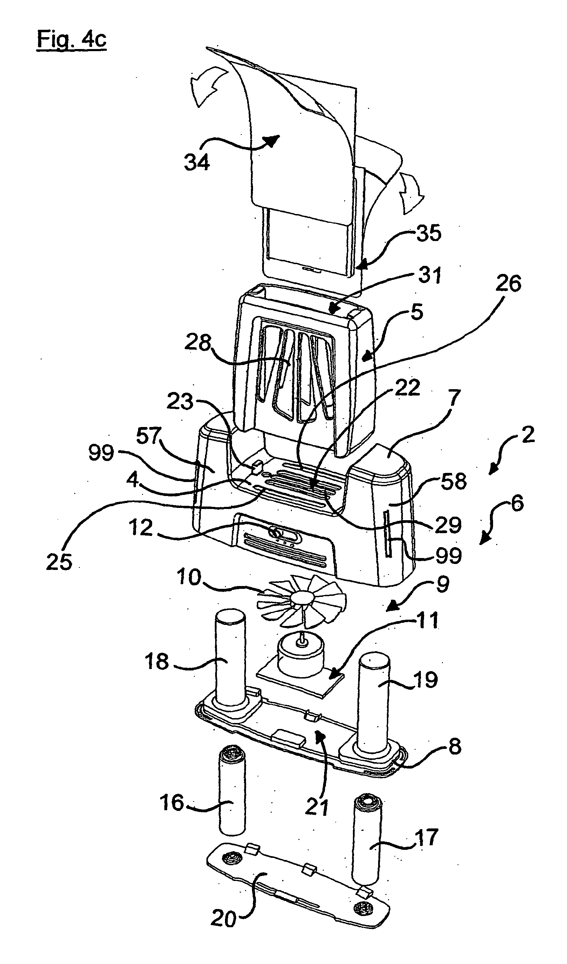

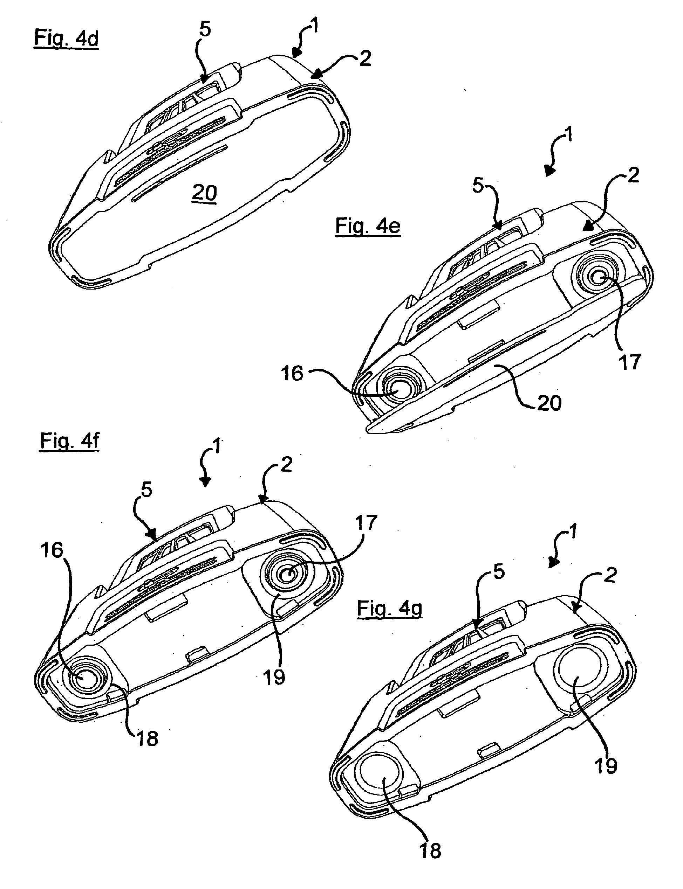

In der Fig. 4 ist schließlich eine weitere alternative Ausführungsform dargestellt, die sich von der Ausführungsvariante gemäß der Fig. 1 insbesondere durch eine andere Ausgestaltung der Andockstation 2 unterscheidet. Die Andockstation 2 weist hier nicht, wie bei den vorherigen Ausführungsformen der Fig. 1 bis 3 horizontal liegende Batterien bzw. Aufnahmefächer auf, sondern, bezogen auf die in der Fig. 4a und 4b gezeigte Betriebs- oder Montageposition, vertikale Aufnahmefächer 18, 19 auf, in denen die Batterien 16, 17 entsprechend stehend bzw. vertikal aufgenommen sind. Auch hier ist wiederum, wie dies insbesondere aus der Fig. 4c ersichtlich ist, im zwischen den beiden Aufnahmefächern 18, 19 für die Batterien 16, 17 angeordneten Bereich ein Montagefreiraum 21 angeordnet, in dem die Ventilatoranordnung 9 montiert und aufgenommen wird (auch hier wiederum gegebenenfalls mit zusätzlichen elektronischen Bauteilen und Komponenten, wie beispielsweise einer Steuer- und/oder Regeleinrichtung 11). Die Aufnahmefächer 18, 19 sind hier Bestandteil des Gehäuseunterteils 8, auf das das Gehäuseoberteil 7 aufgesetzt wird, wobei das Gehäuseoberteil 7 hier eine U-förmige Außenkontur aufweist, mit links-und rechtsseitigen U-Schenkeln 57, 58, in denen die Aufnahmefächer 18, 19 aufgenommen sind. Die Anschlussfläche 4 für die Aufnahmeeinrichtung 5 und damit der Andockbereich 22 sind gegenüber den freien U-Schenkelenden vertieft und abgesenkt angeordnet, so dass im in der Fig. 4a und in der Fig. 4b dargestellten, montierten bzw. angedockten Zustand der Aufnahmeeinrichtung 5 die beiden U-Schenkel 57, 58 die Aufnahmeeinrichtung 5 an den gegenüberliegenden Schmalseiten im Wesentlichen formschlüssig umgreifen und abstützen. Auch hier kann selbstverständlich wiederum eine zusätzliche Fixierung der Aufnahmeeinrichtung 5 über die Rastvorsprünge 23, 24 erfolgen, von denen hier lediglich der Rastvorsprung 23 in der Fig. 4c dargestellt und ersichtlich ist. Ansonsten entspricht der Aufbau und die Funktionsweise derjenigen der Fig. 1.

Die gehäuseseitigen Lufteinlassschlitze 99 sind hier ebenfalls wiederum lediglich beispielhaft und äußerst schematisch eingezeichnet und liegen zum Beispiel im Bereich der U-Schenkel 57, 58.

Lediglich der Vollständigkeit halber sind hier in den Fig. 4d bis 4g Unteransichten dieser Erfindungsvariante auf die Aufstandsfläche 3 und den dort verrastbar gelagerten Deckel 20 gezeigt, die das Abnehmen des Deckels 20 und die Anordnung bzw. Ausgestaltung der Batterien 16, 17 in Verbindung mit den Aufnahmefächern 18, 19 nochmals näher im Detail zeigt.

## Patentansprüche

1. Vorrichtung zur Abgabe von flüchtigen Substanzen, insbesondere von Duftstoffen und/oder Insektiziden,
mit wenigstens einem die wenigstens eine abzugebende Substanz aufweisenden, insbesondere zwischenspeichernden Speichermedium (34, 35), und
mit wenigstens einem Mittel (9) zum Erzeugen wenigstens eines Luftstroms, wobei der wenigstens eine Luftstrom (47, 54) so auf das wenigstens eine Speichermedium (34, 35) gelenkt ist, dass dieser die Abgaberate der abzugebenden Substanz an die Umgebung gegenüber dem nicht-angeströmten Zustand erhöht,
wobei das wenigstens eine Mittel (9) zum Erzeugen wenigstens eines Luftstroms Bestandteil einer Andockstation (2) ist, mit der das wenigstens eine Speichermedium (34, 35) in einem Andockbereich (22) mittelbar über ein das wenigstens eine Speichermedium (34, 35) haltendes und/oder aufnehmendes Bauteil (5) verbindbar ist, dergestalt, dass das, das wenigstens eine Speichermedium (34, 35) halternde und/oder aufnehmende Bauteil (5) in der Betriebsposition an einer Oberseite der Andockstation (2) angedockt ist,
wobei die Andockstation (2) ein ein- oder mehrteiliges Gehäuse (6) aufweist, in dem das wenigstens eine Mittel (9) zur Erzeugung wenigstens eines Luftstroms aufgenommen ist,
wobei das Gehäuse (6) wenigstens eine Luftauslassöffnung (25, 26, 29) und/oder wenigstens eine Lufteinlassöffnung (99) aufweist, und
wobei das wenigstens eine Speichermedium (34, 35) an und/oder in einer Halte- und/oder Aufnahmeeinrichtung (5) gehaltert, insbesondere in einem Aufnahmeraum (31) einer Halte- und/oder Aufnahmeeinrichtung (5) aufgenommen ist, die mit der Andockstation (2) verbunden ist,
**dadurch gekennzeichnet, dass** das wenigstens eine Speichermedium (34, 35) einen definierten Membranbereich (41) aufweist, über den die abzugebende und/oder zu verflüchtigende Substanz vom Speichermedium (34, 35) abgebbar ist und welcher Membranbereich (41) von dem wenigstens einen Luftstrom anströmbar und/oder umströmbar ist,
dass das Speichermedium (34, 35) ein Behältnis ist, in dem die wenigstens eine abzugebende und/oder zu verflüchtigende Substanz, bevorzugt in Form einer Flüssigkeit und/oder eines Gels, aufgenommen ist, wobei ein definierter Behältniswandbereich als Membranbereich (41) ausgebildet ist,
dass das wenigstens eine Speichermedium (34, 35) plättchenförmig und/oder rechteckförmig ausgebildet ist und über eine aufnahmeraumseitige Zugangsöffnung (46) in die Halte- und/oder Aufnahmeeinrichtung (5) einsetzbar, insbesondere mittels einer Art Führungskulissenanordnung geführt einsteckbar und/oder positionierbar ist,
dass ein Aufnahmeraum (31) der Halte- und/oder Aufnahmeeinrichtung (5) wenigstens einen Einsteckschlitz (32, 33) mit beabstandet gegenüberliegenden Führungsnuten (36, 37) aufweist, in denen das plättchenförmige und/oder rechteckförmige Speichermedium (34, 35) mit randseitig gegenüberliegenden Randplattenbereichen (38, 39) eingreift und geführt in die vorgegebene Speichermediumposition überführbar und/oder dort abstützbar und/oder haltbar ist,
dass die Halte- und/oder Aufnahmeeinrichtung (5) im angedockten Betriebszustand zusammen mit wenigstens einem darin eingesetzten Speichermedium (34, 35) turmartig von der ein Basisteil ausbildenden Andockstation (2) wegragt, dergestalt, dass die wenigstens eine Luftauslassöffnung (25, 26, 29) und das wenigstens eine Speichermedium (34, 35) so angeordnet und/oder einander so zugeordnet sind, dass der wenigstens eine Luftstrom (47, 54) das wenigstens eine Speichermedium (34, 35) entlang einer definierten Anströmfläche des Speichermediums (34, 35) an- und/oder umströmt.

2. Vorrichtung nach Anspruch 1, **dadurch gekennzeichnet, dass** die Andockstation (2) als Standgerät mit einer Aufstandsfläche (3) und/oder einer Anschlussfläche (4) für das wenigstens eine Speichermedium (34, 35) oder für die dieses gegebenenfalls aufnehmende Bauteil (5) ausgebildet ist.

3. Vorrichtung nach Anspruch 1 oder 2, **dadurch gekennzeichnet, dass** die Halte- und/oder Aufnahmeeinrichtung (5) in wenigstens einem definierten Außenwandbereich wenigstens eine Lufteinlassöffnung (27, 28), insbesondere wenigstens eine seitliche Lufteinlassöffnung, aufweist, über die wenigstens ein über wenigstens eine andockstationsseitige Luftauslassöffnung (25, 26) ausströmender Luftstrom (47) von der Außenseite der Halte- und/oder Aufnahmeeinrichtung (5) her in diese ein- und/oder ausströmt.

4. Vorrichtung nach einem der Ansprüche 1 bis 3, **dadurch gekennzeichnet, dass** an der Andockstation (2) mehrere, vorzugsweise voneinander beabstandete Luftauslassöffnungen (25, 26), insbesondere Luftauslassschlitze, im Andockbereich (22) um die Halte- und/oder Aufnahmeeinrichtung (5) herum angeordnet und/oder jeweils halte- und/oder aufnahmeeinrichtungsseitige Lufteinlassöffnungen (27, 28) aufweisenden Halte- und/oder Aufnahmeeinrichtungs- Wandbereichen zugeordnet sind, insbesondere so zugeordnet sind, dass die aus den andockstationsseitigen Luftauslassöffnungen (25, 26) austretenden Luftströme (47) auf definiertem, im Wesentlichen geradlinigen Strömungsweg zu den Lufteinlassöffnungen (27, 28) strömen.

5. Vorrichtung nach einem der Ansprüche 1 bis 4, **dadurch gekennzeichnet, dass** die Halte- und/oder Aufnahmeeinrichtung (5) im Andockbereich (22) wenigstens eine Lufteinlassöffnung (48, 49) aufweist, die im angedockten Zustand wenigstens eine andockstationsseitige Luftauslassöffnung (29) so überdeckt, dass dadurch wenigstens ein Luftstrom (54) in das Innere der Halte- und/oder Aufnahmeeinrichtung (5) bevorzugt unmittelbar in einen Aufnahmeraum (31) der Halte- und/oder Aufnahmeeinrichtung (5) einströmt.

6. Vorrichtung nach Anspruch 5, **dadurch gekennzeichnet, dass** die wenigstens eine Lufteinlassöffnung (48, 49) der Halte- und/oder Aufnahmeeinrichtung (5) so ausgebildet ist, dass der wenigstens eine Luftstrom (54) das wenigstens eine Speichermedium (34, 35) von unten her entlang einer definierten Wegstrecke umströmt und/oder so ausgebildet ist, dass diese mit wenigstens einem halte- und/oder aufnahmeeinrichtungsseitigen Strömungskanal (51, 52) strömungsverbunden ist, mittels dem der wenigstens eine Luftstrom (54) zum Aufnahmeraum (31) geführt ist.

7. Vorrichtung nach einem der Ansprüche 1 bis 6, **dadurch gekennzeichnet, dass** wenigstens eine halte- und/oder aufnahmeeinrichtungsseitige Abströmöffnung durch wenigstens eine Zugangsöffnung (46) des Aufnahmeraums (31), über die das wenigstens eine Speichermedium (34, 35) in den Aufnahmeraum (31) einsetzbar ist, gebildet ist und/oder in einer Doppelfunktion durch die das Einströmen des wenigstens einen Luftstroms in den Aufnahmeraum (31) ermöglichende wenigstens eine seitliche Lufteinlassöffnung (27, 28) gebildet ist.

8. Vorrichtung nach einem der Ansprüche 1 bis 7, **dadurch gekennzeichnet, dass** ein Membranbereich (41) eines Speichermediums der wenigstens einen Lufteinlassöffnung (27, 28) so zugewandt ist, dass der wenigstens eine, über diese in die Aufnahmeeinrichtung einströmende Luftstrom (47) auf den Membranbereich (41) trifft.

9. Vorrichtung nach einem der Ansprüche 1 bis 8, **dadurch gekennzeichnet, dass** der Membranbereich (41) mit einer abnehmbaren Abdeckung (42), insbesondere mit einer Abziehfolie, abgedeckt und/oder abgedichtet ist.

10. Vorrichtung nach einem der Ansprüche 1 bis 9, **dadurch gekennzeichnet, dass** ein Membranbereich (41) des Speichermediums (34, 35) wenigstens bereichsweise durch wenigstens eine der beiden großflächigen Seitenflächen gebildet ist.

11. Vorrichtung nach einem der Ansprüche 1 bis 10, **dadurch gekennzeichnet, dass** der Aufnahmeraum (31) mehrere, insbesondere zwei im Wesentlichen gleich ausgebildete und/oder voneinander quer zur Einsteckrichtung beabstandete Einsteckschlitze (32, 33) aufweist.

12. Vorrichtung nach einem der Ansprüche 1 bis 11, **dadurch gekennzeichnet, dass** wenigstens eine Steuer- und/oder Regeleinrichtung (11) vorgesehen ist, mittels der das wenigstens eine Mittel (9) zum Erzeugen wenigstens eines Luftstroms in Abhängigkeit von definierten Betriebsparametern ansteuerbar ist, insbesondere zur Einstellung der Geschwindigkeit und/oder der Menge des zum wenigstens einen Speichermedium geführten wenigstens einen Luftstroms, wobei bevorzugt vorgesehen ist, dass die Steuer- und/oder Regeleinrichtung (11) eine Speichereinrichtung aufweist, in der wenigstens ein vorzugsweise mehrere unterschiedliche vorprogrammierte und/oder auswählbare Betriebsmodi und/oder Betriebszyklen abgelegt ist bzw. sind.

13. Vorrichtung nach einem der Ansprüche 1 bis 12, **dadurch gekennzeichnet, dass** wenigstens eine Leuchteinrichtung (56), vorzugsweise wenigstens eine Leuchtdiode vorgesehen ist, mittels der der jeweilige Betriebszustand und/oder Betriebsmodus anzeigbar und/oder die Vorrichtung (1), insbesondere die Andockstation (2) und/oder das wenigstens eine Speichermedium (34, 35) und/oder wenigstens ein das wenigstens eine Speichermedium (34, 35) aufnehmende Bauteil (5) beleuchtbar und/oder ausleuchtbar ist.

## Claims

1. Device for dispensing volatile substances, in particular fragrances and/or insecticides,
having at least one storage medium (34, 35) having the at least one substance that is to be dispensed, in particular temporarily, and
having at least one means (9) for generating at least one air flow, wherein the at least one air flow (47, 54) is directed towards the at least one storage medium (34, 35) in such a way that this increases the rate of dispensation of the substance that is to be dispensed to the environment relative to the state in which there is no flow against the storage medium,
wherein the at least one means (9) for generating at least one air flow is a component of a docking station (2), to which the at least one storage medium (34, 35) can be connected in a docking region (22) indirectly via a component (5) holding and/or receiving a storage medium (34, 35), in such a way that the component (5) holding and/or receiving the at least one storage medium (34, 35) is docked in the operating position on the upper side of the docking station (2),
wherein the docking station (2) has a single-part or multipart housing (6), in which the at least one means (9) for generating at least one air flow is received,
wherein the housing (6) has at least one air outlet opening (25, 26, 29) and/or at least one air inlet opening (99), and
wherein the at least one storage medium (34, 35) is supported at and/or in a support and/or receiving device (5), in particular in a receiving space (31) of a support and/or receiving device (5), which is connected to the docking station (2),
**characterised in that**, the at least one storage medium (34, 35) has a defined membrane region (41), via which the substance to be dispensed and/or volatilised can be dispensed from the storage medium (34, 35) and that said membrane region (41) can be flowed against and/or around by the at least one air flow,
that the storage medium (34, 35) is a container in which the at least one substance to be dispensed and/or volatilised, preferably in the form of a liquid and/or a gel, is received, wherein a defined container wall region is designed as the membrane region (41),
that the at least one storage medium (34, 35) is designed as platelets and/or rectangles and via which a receiver-space-side access opening (46) can be introduced into the support and/or receiving device (5), in particular inserted and/or positioned in a controlled manner by means of a type of guiding coulisse arrangement,
that a receiving space (31) of the support and/or receiving device (5) has at least one insertion slit (32, 33) with guide slots (36, 37) opposite at a distance, in which the platelet-shaped and/or rectangular storage medium (34, 35) engages with edge plate regions (38, 39) located opposite at the edge and that can be transferred in a controlled manner into the predetermined storage medium position and/or braced and/or supported there,
that the support and/or receiving device (5), in the docked operating state, together with at least one storage medium (34, 35) introduced therein, protrudes like a tower from the docking station (2) that forms a core section, in such a way that the at least one air outlet opening (25, 26, 29) and the at least one storage medium (34, 35) are arranged in such a way and/or assigned to each other in such a way that the at least one air flow (47, 54) flows against and/or around the at least one storage medium (34, 35) along a defined flow surface of the storage medium (34, 35).

2. Device according to claim1, **characterised in that**, the docking station (2) is designed as a floor-mounted device having a contact surface (3) and/or a mounting surface (4) for the at least one storage medium (34, 35) or for the component (5) receiving this, if necessary.

3. Device according to claim 1 or 2, **characterised in that**, the support and/or receiving device (5), in at least one defined outer wall region, has at least one air inlet opening (27, 28), in particular at least one lateral air inlet opening, via which at least one air flow (47) flowing out via at least one docking-station-side air outlet opening (25, 26) flows in and/or out from the outer side of the support and/or receiving device (5) into this.

4. Device according to one of claims 1 to 3, **characterised in that**, at the docking station (2), several air outlet openings (25, 26) that are preferably at a distance to one another, in particular air outlet slits, are arranged in the docking region (22) around the support and/or receiving device (5) and/or are assigned to support and/or receiving device wall regions having respective support and/or receiving-device-side air inlet openings (27, 28), in particular being assigned in such a way that the air flows (47) departing from the docking-station-side air outlet openings (25, 26) flow on a defined, essentially straight flow path to the air inlet openings (27, 28).

5. Device according to one of claims 1 to 4, **characterised in that**, the support and/or receiving device (5) has at least one air inlet opening (48, 49) in the docking region (22), which, in the docked state, covers at least one docking-station-side air outlet opening (29) in such a way that at least one air flow (54) thus flows into the interior of the support and/or receiving device (5), and preferably directly into a receiving space (31) of the support and/or receiving device (5).

6. Device according to claim 5, **characterised in that**, the at least one air inlet opening (48, 49) of the support and/or receiving device (5) is designed in such a way that the at least one air flow (54) flows around the at least one storage medium (34, 35) from below along a defined distance and/or is designed in such a way that this is flow-connected to at least one support and/or receiving-device-side flow channel (51, 52), by means of which the at least one air flow (54) is guided to the receiving space (31).

7. Device according to one of claims 1 to 6, **characterised in that**, at least one support and/or receiving-device-side flow-off opening is formed by at least one access opening (46) of the receiving space (31), via which the at least one storage medium (34, 35) can be introduced into the receiving space (31) and/or is formed in a dual function by the at least one lateral air inlet opening (27, 28), which enables the in-flow of the at least one air flow into the receiving space (31).

8. Device according to one of claims 1 to 7, **characterised in that**, a membrane region (41) of a storage medium of the at least one air inlet opening (27, 28) is turned in such a way that the at least one air flow (47) flowing into the receiving device via this opening encounters the membrane region (41).

9. Device according to one of claims 1 to 8, **characterised in that**, the membrane region (41) is covered and/or sealed off by a removable covering (42), in particular with a detachable foil.

10. Device according to one of claims 1 to 9, **characterised in that**, a membrane region (41) of the storage medium (34, 35) is formed at least in certain regions by at least one of both extensive lateral surfaces.

11. Device according to one of claims 1 to 10, **characterised in that**, the receiving space (31) has several insertion slits (32, 33), in particular two that are designed essentially identically and/or at a distance from each other at right angles to the direction of insertion.

12. Device according to one of claims 1 to 11, **characterised in that**, at least one control and/or regulating device (11) is provided, by means of which the at least one means (9) for generating at least one air flow can be activated depending on defined operating parameters, in particular for adjusting the speed and/or the amount of the at least one air flow guided to at least one storage medium, wherein provision is preferably made for the control and/or regulating device (11) to have a storage device, in which at least one, preferably several different pre-programmed and/or selectable operating modes and/or operating cycles is/are removed.

13. Device according to one of claims 1 to 12, **characterised in that**, at least one lighting device (56), preferably at least one LED, is provided, by means of which the respective operating state and/or operating mode can be displayed and/or the device (1), in particular the docking station (2) and/or the at least one storage medium (34, 35) and/or at least one component (5) receiving the at least one storage medium (34, 35), can be illuminated and/or lit up.

## Revendications

1. Dispositif de diffusion de substances volatiles, en particulier de parfums et/ou d'insecticides,
comportant au moins un support de stockage (34, 35) présentant, en particulier stockant de façon intermédiaire, au moins une substance à diffuser, et
comportant au moins un moyen (9) de génération d'au moins un courant d'air, l'au moins un courant d'air (47, 54) étant orienté sur l'au moins un support de stockage (34, 35) de sorte que celui-ci augmente le taux de diffusion de la substance à diffuser dans l'environnement par rapport à l'état non parcouru par le courant d'air,
dans lequel l'au moins un moyen (9) de génération d'au moins un courant d'air est un composant d'un poste de fixation (2) auquel l'au moins un support de stockage (34, 35) peut être relié dans une zone de fixation (22), indirectement par un élément (5) tenant et/ou recevant l'au moins un support de stockage (34, 35), de telle sorte que l'élément (5) tenant et/ou recevant l'au moins un support de stockage (34, 35) soit fixé, dans la position de fonctionnement, sur un côté supérieur du poste de fixation (2),
dans lequel le poste de fixation (2) présente un boîtier à une ou plusieurs parties (6), dans lequel l'au moins un moyen (9) de génération d'au moins un courant d'air est reçu,
dans lequel le boîtier (6) présente au moins une ouverture de sortie d'air (25, 26, 29) et/ou au moins une ouverture d'entrée d'air (99), et
dans lequel l'au moins un support de stockage (34, 35) est conservé sur et/ou dans un système de retenue et/ou de réception (5), en particulier dans un espace de réception (31) d'un système de retenue et/ou de réception (5) qui est relié au poste de fixation (2),
**caractérisé en ce que** l'au moins un support de stockage (34, 35) présente une zone de membrane définie (41) par laquelle la substance à diffuser et/ou à volatiliser peut être diffusée par le support de stockage (34, 35) et ladite zone de membrane (41) peut être parcourue et/ou contournée par l'au moins un courant d'air,
**en ce que** le support de stockage (34, 35) est un récipient dans lequel l'au moins une substance à diffuser et/ou à volatiliser, est reçue de préférence sous la forme d'un liquide et/ou d'un gel, une zone de paroi de récipient étant conçue comme une zone de membrane (41),
**en ce que** l'au moins un support de stockage (34, 35) est conçu sous forme de plaquette ou de rectangle et peut être inséré par une ouverture d'accès (46) du côté de l'espace de réception, dans le système de retenue et/ou de réception (5), en particulier peut être fiché et/ou positionné au moyen d'une sorte d'aménagement de guidage coulissant,
**en ce qu'**un espace de réception (31) du système de retenue et/ou de réception (5) présente au moins une fente d'insertion (32, 33) comportant des rainures de guidages opposées distantes (36, 37) dans lesquelles le support de stockage (34, 35) sous forme de plaquette et/ou de rectangle se met en prise avec des zones de plaque périphériques (38, 39) à bords opposés et, conduit dans la position du support de stockage prédéterminée, peut être transféré et/ou appuyé et/ou retenu,
**en ce que** le système de retenue et/ou de réception (5) dépasse à l'état de fonctionnement fixé, conjointement avec au moins un support de stockage (34, 35) qui y est inséré, comme une tour, du poste de fixation (2) formant une partie de base, de telle sorte que l'au moins une ouverture d'entrée d'air (25, 26, 29) et l'au moins un support de stockage (34, 35) soient disposés et/ou agencés l'un par rapport à l'autre de sorte que l'au moins un courant d'air (47, 54) parcoure et/ou contourne l'au moins un support de stockage (34, 35) le long d'une surface de parcours définie du support de stockage (34, 35).

2. Dispositif selon la revendication 1, **caractérisé en ce que** le poste de fixation (2) est conçu comme un appareil sur pied doté d'une surface de contact au sol (3) et/ou d'une surface de raccordement (4) pour l'au moins un support de stockage (34, 35) ou pour laquelle cet élément de réception (5) éventuel est conçu.

3. Dispositif selon la revendication 1 ou 2, **caractérisé en ce que** le système de retenue et/ou de réception (5) présente, dans au moins une zone de paroi externe définie, au moins une ouverture d'entrée d'air (27, 28), en particulier au moins une ouverture d'entrée d'air latérale, par laquelle au moins un courant d'air (47) sortant par au moins une ouverture de sortie d'air (25, 26) du côté du poste de fixation en venant du côté extérieur du système de retenue et/ou de réception (5) entre dans celle-ci et/ou en sort.

4. Dispositif selon l'une des revendications 1 à 3, **caractérisé en ce que**, sur le poste de fixation (2) plusieurs ouvertures de sortie d'air (25, 26), de préférence, à distance les unes des autres, en particulier des fentes de sortie d'air sont disposées dans la zone de fixation (22), tout autour du système de retenue et/ou de réception et/ou sont affectées respectivement aux zones de paroi du système de retenue et/ou de réception présentant des ouvertures d'entrée d'air (27, 28) du côté du système de retenue et/ou de réception, en particulier, sont affectées de telle sorte que les courants d'air (47) sortant des ouvertures de sortie d'air (25, 26) du côté du poste de fixation s'écoulent sur une voie d'écoulement définie, essentiellement linéaire, jusqu'aux ouvertures d'entrée d'air (27, 28).

5. Dispositif selon l'une des revendications 1 à 4, **caractérisé en ce que** le système de retenue et/ou de réception (5) présente dans la zone de fixation (22) au moins une ouverture d'entrée d'air (48, 49) qui recouvre à l'état fixé au moins une ouverture de sortie d'air (29) du côté du poste de fixation de telle sorte qu'ainsi, au moins un courant d'air (54) entre dans l'intérieur du système de retenue et/ou de réception (5), de préférence directement dans un espace de réception (31) du système de retenue et/ou de réception (5).

6. Dispositif selon la revendication 5, **caractérisé en ce que** l'au moins une ouverture d'entrée d'air (48, 49) du système de retenue et/ou de réception (5) est conçue de telle sorte que l'au moins un courant d'air (54) contourne l'au moins un support de stockage (34, 35) à partir du bas en passant le long d'une voie définie et/ou est conçue de telle sorte que celle-ci soit en relation d'écoulement avec au moins un canal d'écoulement (51, 52) du côté du système de retenue et/ou de réception, au moyen duquel l'au moins un courant d'air (54) est acheminé jusqu'à l'espace de réception (31).

7. Dispositif selon l'une des revendications 1 à 6, **caractérisé en ce que** l'au moins une ouverture d'écoulement du côté du dispositif de retenue et/ou de réception est formée par au moins une ouverture d'admission (46) de l'espace de réception (31) par laquelle l'au moins un support de stockage (34, 35) peut être inséré dans l'espace de réception (31) et/ou, dans une double fonction, est formée par l'au moins une ouverture d'entrée d'air (27, 28) latérale permettant l'entrée de l'au moins un courant d' air dans l'espace de réception (31).

8. Dispositif selon l'une des revendications 1 à 7, **caractérisé en ce qu'**une zone de membrane (41) d'un support de stockage de l'au moins une ouverture d'entrée d'air (27, 28) est orientée de telle sorte que l'au moins un courant d'air (47) entrant par celle-ci dans le système de réception arrive sur la zone de membrane (41).

9. Dispositif selon l'une des revendications 1 à 8, **caractérisé en ce que** la zone de membrane (41) est recouverte et/ou étanchéifiée par un revêtement amovible (42), en particulier, une feuille à retirer.

10. Dispositif selon l'une des revendications 1 à 9, **caractérisé en ce qu'**une zone de membrane (41) du support de stockage (34, 35) est formée au moins partiellement par au moins l'une des deux surfaces latérales larges.

11. Dispositif selon l'une des revendications 1 à 10, **caractérisé en ce que** l'espace de réception (31) présente plusieurs, en particulier deux fentes d'insertion (32, 33) formées essentiellement de façon identique et/ou distantes l'une de l'autre perpendiculairement à la direction d'insertion.

12. Dispositif selon l'une des revendications 1 à 11, **caractérisé en ce qu'**au moins un système de commande et/ou de régulation (11) est prévu, au moyen duquel l'au moins un moyen (9) de génération d'au moins un courant d'air peut être commandé en fonction de paramètres de fonctionnement définis, en particulier pour ajuster la vitesse et/ou la quantité d'au moins un courant d'air conduit à au moins un support de stockage, dans lequel il est prévu de préférence que le système de commande et/ou de régulation (11) présente une mémoire dans laquelle au moins un, de préférence plusieurs modes de fonctionnement et/ou cycles de fonctionnement préprogrammés différents et/ou sélectionnables est ou sont enregistré(s).

13. Dispositif selon l'une des revendications 1 à 12, **caractérisé en ce qu'**au moins un système d'éclairage (56), de préférence au moins une diode luminescente est prévu(e), au moyen duquel/de laquelle
l'état de fonctionnement et/ou le mode de fonctionnement respectif peut être indiqué et/ou au moyen duquel/de laquelle le dispositif (1), en particulier le poste de fixation (2) et/ou l'au moins un support de stockage (34, 35) et/ou au moins un élément (5) recevant l'au moins un support de stockage (34, 35) peut être éclairé et/ou éteint.
